# EUROPEAN PATENT APPLICATION

(11) **EP 4 785 953 A1**
(43) Date of publication of application: **05.08.2026**
(21) Application number: 24871027.9
(22) Date of filing: 27.09.2024
(51) Int. Cl.: A61K 39/395, C07K 16/28, C07K 16/46, A61P 35/00

(54) **USE OF BISPECIFIC ANTIBODY IN PREPARATION OF DRUG FOR TREATING CANCERS**

(30) Priority: 28.09.2023 CN 202311283448
(71) Applicant: Shanghai Hansoh Biomedical Co., Ltd., Shanghai 201203 (CN); Changzhou Hansoh Pharmaceutical Co., Ltd., Xinbei District Changzhou Jiangsu 213001 (CN); Biontech (Zhuhai) Pharmaceuticals R&D Co., Ltd, Zhuhai, Guangdong 519080 (CN)
(72) Inventor: MA, Xiaoying, Shanghai 201203 (CN); QIAN, Hui, Shanghai 201203 (CN); ZHOU, Yuanfeng, Shanghai 201203 (CN); CHEN, Lili, Shanghai 201203 (CN)
(74) Representative: Maiwald GmbH
(86) International application number: PCT/CN2024/121938
(87) International publication number: WO 2025/067492

(57) **Abstract**

Provided is a use of a bispecific antibody in preparation of a drug for treating cancers, and in particular a use in preparation of a drug for treating advanced solid tumors.

## Description

The present application claims priority to Chinese Patent Application No. 202311283448.4 filed on September 28, 2023, which is incorporated in its entirety herein by reference.

### Technical Field

The present invention belongs to the field of medicine and relates to a use of a bispecific antibody in the manufacture of a medicament for treating cancer, wherein the bispecific antibody is a drug that inhibits the human epidermal growth factor receptor and the tyrosine kinase Met as dual targets. Based on its unique structure and novel mechanism of action, the bispecific antibody has the potential to achieve clinical benefits that are difficult to obtain with monotherapy or combination therapy.

### Background of the Disclosure

EGFR is a member of the human epidermal growth factor receptor (HER) family, and is widely distributed on the surface of tissues or cells derived from epithelium, mesenchyme, and neurons. Under physiological conditions, EGFR can promote cell differentiation, proliferation, migration, and survival after binding to its ligands, such as epidermal growth factor EGF. Under pathological conditions, EGFR, as an oncogene, can promote the tumorigenesis and development of tumors through mechanisms such as gene mutation and upregulated expression. Common EGFR gene mutation sites are exons 18-21, mainly exon 19 deletion (Exon 19del), exon 21 L858R point mutation (L858R), exon 20 insertion mutation (Exon 20ins), etc. EGFR mutations or overexpression show a high incidence in NSCLC (50% mutation), head and neck cancer (80% overexpression), colon cancer (25% mutation), and breast cancer (11% overexpression/mutation), and multiple EGFR-targeting drugs have demonstrated clinical effectiveness in the above indications.

The *MET* gene is a proto-oncogene and encodes the cellular mesenchymal-epithelial transition factor (c-MET), also known as the hepatocyte growth factor receptor (HGFR). c-MET/HGFR also belongs to the receptor tyrosine kinase (RTK) family. It is a transmembrane tyrosine kinase receptor that, upon binding to its ligand hepatocyte growth factor (HGF), can induce MET dimerization and phosphorylate its substrate, thereby activating downstream signaling pathways involved in the regulation of cell proliferation, differentiation, migration, and angiogenesis.

The c-MET pathway is closely related to EGFR resistance. MET gene expression bypasses the repressed EGFR phosphorylation kinase pathway, providing a bypass route by activating ERBB3 and the PI3K/AKT pathway downstream of MET. MET activates and promotes downstream signal transduction through this bypass pathway, avoiding apoptosis induced by tyrosine kinase inhibitors (TKIs) of EGFR, while promoting tumor cell proliferation, ultimately leading to EGFR TKI resistance. Therefore, simultaneously inhibiting EGFR and c-MET pathways to overcome EGFR TKI resistance caused by MET pathway activation is highly necessary, which provides a theoretical basis for combining c-MET pathway inhibition with EGFR pathway inhibition in the treatment of advanced solid tumors.

EGFR Exon 20ins, due to its unique mutation structure, is not sensitive to the existing first to third generation EGFR TKIs, and chemotherapy and immunotherapy are also ineffective. The first-line standard treatment recommended by the 2022 CSCO guidelines remains platinum-based chemotherapy. There is currently no standard treatment for patients with advanced solid tumors that are resistant to EGFR-targeted therapies caused by c-MET pathway activation. Therefore, finding effective therapeutic methods is an urgent clinical need.

### Summary of the Invention

The present invention provides use of a bispecific antibody specifically binding to c-Met and EGFR in the manufacture of a medicament for preventing or treating cancer.

The present invention also provides use of a pharmaceutical composition of a bispecific antibody specifically binding to c-Met and EGFR in the manufacture of a medicament for preventing or treating cancer.

The present invention also provides use of a bispecific antibody specifically binding to c-Met and EGFR, or a pharmaceutical composition of the bispecific antibody, in combination with an EGFR inhibitor, in the manufacture of a medicament for preventing or treating a cancer.

In some embodiments, the EGFR inhibitor is selected from erlotinib, gefitinib, befotertinib, osimertinib, almonertinib, furmonertinib, oritinib, Rezetinib, Avitinib, olmutinib, and rociletinib; and preferably is almonertinib or osimertinib.

In some embodiments, the bispecific antibody or the bispecific antibody pharmaceutical composition, and the EGFR inhibitor are each comprised in different formulations as active ingredients, and are administered simultaneously, concurrently, sequentially, continuously, alternately, or separately.

In some embodiments, the bispecific antibody comprises a first antibody or antigen-binding fragment against the first target c-Met, and a second antibody or antigen-binding fragment against the second target EGFR; wherein the first antibody or antigen-binding fragment comprises an HCDR1 as set forth in SEQ ID NO: 01, an HCDR2 as set forth in SEQ ID NO: 02, an HCDR3 as set forth in SEQ ID NO: 03; and an LCDR1 as set forth in SEQ ID NO: 04, an LCDR2 as set forth in SEQ ID NO: 05, an LCDR3 as set forth in SEQ ID NO: 06; wherein the second antibody or antigen-binding fragment comprises an HCDR1 as set forth in SEQ ID NO: 09, an HCDR2 as set forth in SEQ ID NO: 10, an HCDR3 as set forth in SEQ ID NO: 11; and an LCDR1 as set forth in SEQ ID NO: 12, an LCDR2 as set forth in SEQ ID NO: 13, an LCDR3 as set forth in SEQ ID NO: 14.

In some embodiments, the bispecific antibody comprises a first antibody or antigen-binding fragment against the first target c-Met and a second antibody or antigen-binding fragment against the second target EGFR, the first antibody or antigen-binding fragment comprising a heavy chain variable region as set forth in SEQ ID NO: 07 and a light chain variable region as set forth in SEQ ID NO: 08; the second antibody or antigen-binding fragment comprising a heavy chain variable region as set forth in SEQ ID NO: 15, and a light chain variable region as set forth in SEQ ID NO: 16.

In some embodiments, in the bispecific antibody, the c-Met binding domain is a Fab region formed by a first heavy chain and a first light chain, the EGFR binding domain is a Fab region formed by a second heavy chain and a second light chain, and the first heavy chain and the second heavy chain bind to each other to form a heterodimeric Fc region.

In some embodiments, the bispecific antibody comprises a first antibody or antigen-binding fragment against the first target c-Met, comprising a heavy chain as set forth in SEQ ID NO: 21, and a light chain as set forth in SEQ ID NO: 22; and a second antibody or antigen-binding fragment against the second target EGFR, comprising a heavy chain as set forth in SEQ ID NO: 23, and a light chain as set forth in SEQ ID NO: 24.

In some embodiments, the cancer is a cancer expressing c-Met or/and EGFR.

In some embodiments, the cancer is a cancer expressing c-Met.

In some embodiments, the cancer is a cancer expressing EGFR.

In some embodiments, the cancer is a cancer expressing c-Met and EGFR.

In some embodiments, the cancer expressing c-MET or/and EGFR is mediated by wild-type EGFR, EGFR activating mutations, EGFR gene amplification, increased level of circulating HGF, wild-type c-Met, c-Met activating mutations, c-Met gene amplification, MET gene amplification, or mutant KRAS, preferably mediated by EGFR activating mutations, or an MET gene amplification.

Exemplary EGFR activating mutations that can be associated with cancer include point mutations, deletion mutations, insertion mutations, inversions, or gene amplifications that cause an increase in at least one biological activity of EGFR, (such as increased tyrosine kinase activity, receptor homodimer and heterodimer formation, enhanced ligand binding, etc). The mutations can be located in any portion of the *EGFR* gene or regulatory regions associated with the *EGFR* gene, and include mutations in exon 18, 19, 20, or 21, or mutations in the kinase domain. Other examples of EGFR activating mutations are known in the art (see, e.g., U.S. Patent Publication US2005/0272083). Information on EGFR and other ErbB receptors, including receptor homodimers and heterodimers, receptor ligands, autophosphorylation sites, and signaling molecules involved in ErbB-mediated signaling, is known in the art (see, e.g., Hynes and Lane,Nature Reviews Cancer 5:341-354,2005).

In some embodiments, the cancer is a cancer expressing EGFR or/and c-Met mediated by EGFR activating mutations. In some embodiments, the EGFR activating mutations comprise at least one mutation selected from the group consisting of G719X (X is any amino acid), L861X (X is any amino acid).

In some embodiments, the EGFR activating mutations comprise at least one mutation selected from the group consisting of: an L718Q, L718V, G719A, G719S, G719C, G719D, L858R, L858P, L861Q, G724S, E746K, L747S, E749Q, A750P, A755V, V765M, C797S, S768I, or T790M substitutions; deletion of E746-A750; deletion of R748-P753; insertion of Ala (A) between M766 and A767; an insertions of Ser, Val, and Ala (SVA) between S768 and V769; insertion of Asn and Ser (NS) between P772 and H773; insertion(s) of one or more amino acids between D761 and E762, between A763 and Y764, between Y764 and Y765, between M766 and A767, between A767 and V768, between S768 and V769, between V769 and D770, between D770 and N771, between N771 and P772, between P772 and H773, between H773 and V774, or between V774 and C775; deletion(s) of one or more amino acids in EGFR exon 19; or insertion(s) of one or more amino acids in EGFR exon 19; one or more deletions in EGFR exon 20; or insertion(s) of one or more amino acids in EGFR exon 20; or any combination thereof.

In some embodiments, the EGFR activating mutations comprise at least one mutation selected from the group consisting of L858R mutation, T790M mutation, L861Q mutation, one or more insertion mutations in EGFR exon 20, and deletion mutation(s) of one or more amino acids in EGFR exon 19. In some embodiments, the cancer is selected from solid tumors, preferably advanced solid tumors.

In some embodiments, the cancer is selected from an epithelial cell carcinoma, breast cancer, ovarian cancer, lung cancer, rectal cancer, anal cancer, prostate cancer, kidney cancer, bladder cancer, head and neck cancer, pharyngeal cancer, nasal cancer, pancreatic cancer, skin cancer, oral cancer, tongue cancer, esophageal cancer, vaginal cancer, endometrial cancer, cervical cancer, spleen cancer, testicular cancer, gastric cancer, thymus cancer, colon cancer, thyroid cancer, liver cancer or melanoma, preferably lung cancer or liver cancer.

In some embodiments, the cancer is selected from a non-small cell lung cancer (NSCLC), a lung adenocarcinoma, a lung squamous cell carcinoma, a large cell lung cancer, a small cell lung cancer, a pulmonary sarcomatoid carcinoma, and a hepatocellular carcinoma (HCC).

In a preferred embodiment, the cancer is selected from a non-squamous non-small cell lung cancer. In a preferred embodiment, the cancer is selected from a locally advanced or metastatic non-squamous non-small cell lung cancer.

In a preferred embodiment, the cancer is selected from a locally advanced or metastatic non-small cell lung cancer carrying the EGFR activating mutations comprising at least one mutation selected from the group consisting of L858R mutation, T790M mutation, L861Q mutation, C797S mutation, one or more insertion mutations in EGFR exon 20, and deletion mutation(s) of one or more amino acids in EGFR exon 19.

In a preferred embodiment, the cancer is selected from a locally advanced or metastatic non-squamous non-small cell lung cancer carrying at least one mutation selected from the group consisting of L858R mutation, one or more insertion mutations in EGFR exon 20, and deletion mutation(s) of one or more amino acids in EGFR exon 19.

In a preferred embodiment, the cancer is selected from a locally advanced or metastatic non-squamous non-small cell lung cancer comprising deletion mutation(s) of one or more amino acids in EGFR exon 19, or an L858R mutation.

In a preferred embodiment, the cancer is selected from a locally advanced or metastatic non-small cell lung cancer comprising one or more insertion mutations in EGFR exon 20.

In some embodiments, the EGFR inhibitor is administered in a dose of 10 to 500 mg, preferably 10 to 200 mg, more preferably 100 mg, 110 mg, 120 mg, 130 mg, 140 mg, 150 mg, 160 mg, 170 mg, 180 mg, 190 mg, or 200 mg.

In some embodiments, the EGFR inhibitor is administered at a frequency of twice a day, once a day, once every two days, or once every three days, preferably once a day, or once every two days.

In a preferred embodiment, the EGFR inhibitor is administered at a dose of 110 mg, and at a frequency of once a day.

The bispecific anti-EGFR/c-Met antibody can be administered in a pharmaceutically acceptable excipient, diluent, or carrier.

"Carrier" refers to a diluent, adjuvant, excipient, or vehicle with which the antibody of the present invention is administered. Such vehicles may be liquids, such as water and oils, including oils derived from petroleum, animals, plants, or synthetic oils, such as peanut oil, soybean oil, mineral oil, or sesame oil. For example, 0.4% saline and 0.3% glycine can be used to formulate the bispecific anti-EGFR/c-Met antibody. These solutions are sterile and generally free of particulate matter. They can be sterilized by conventional, well-known sterilization techniques, such as filtration. For parenteral administration, the carrier may include sterile water, and other excipients may be added to increase solubility or preservation. Suspensions or solutions for injection may also be prepared using aqueous-based carriers together with suitable additives. Suitable vehicles and formulations include other human proteins (e.g., human serum albumin).

In some embodiments, the bispecific antibody composition has a unit concentration of about 1 mg/mL to about 100 mg/mL, preferably about 5 mg/mL to about 50 mg/mL, more preferably about 10 mg/mL to about 30 mg/mL.

In some embodiments, the bispecific antibody pharmaceutical composition has a unit concentration of about 10 mg/mL, about 15 mg/mL, about 20 mg/mL, about 25 mg/mL, or about 30 mg/mL.

In some embodiments, the bispecific antibody pharmaceutical composition is in a specification of 10 mL:10 mg to 10 mL:1000 mg, preferably 10 mL:100 mg to 10 mL:500 mg, more preferably 10 mL:100 mg, 10 mL:200 mg, 10 mL:300 mg, 10 mL:400 mg, or 10 mL:500 mg per vial.

The mode of administration may be any suitable route for delivering the bispecific anti-EGFRxc-Met antibody to a host, such as parenteral administration, for example, intradermal, intramuscular, intraperitoneal, intravenous or subcutaneous, pulmonary, or transmucosal (oral, intranasal, intravaginal, rectal) administration, using a formulation in the form of tablets, capsules, solutions, powders, gels, or granules; contained in syringes, implantable devices, osmotic pumps, cartridges, or a micropumps; or other modes well known in the art, as understood by the skilled person. Site-specific administration can be achieved, for example, by intratumoral, parenteral, intrabronchial, intraabdominal, intracapsular, intrachondral, intracavitary, intracerebellar, intraventricular, intracolonic, intracervical, intragastric, intrahepatic, intracardiac, intraosseous, intrapelvic, intrapericardial, intraperitoneal, intrapleural, intraprostatic, intrapulmonary, intrarectal, intrarenal, intraretinal, intraspinal, intrasynovial, intrathoracic, intrauterine, intravascular, intravesical, intralesional, vaginal, rectal, oral, sublingual, intranasal, or transcutaneous delivery.

In some embodiments, the bispecific anti-EGFR/c-Met antibody or pharmaceutical composition thereof is administered at a dose between about 100 mg and about 5000 mg.

In some embodiments, the bispecific anti-EGFR/c-Met antibody or pharmaceutical composition thereof is administered at a dose between about 400 mg and about 2000 mg.

In some embodiments, the bispecific anti-EGFR/c-Met antibody or pharmaceutical composition thereof is administered at about 400 mg, about 410 mg, about 420 mg, about 430 mg, about 440 mg, about 450 mg, about 460 mg, about 470 mg, about 480 mg, about 490 mg, about 500 mg, about 510 mg, about 520 mg, about 530 mg, about 540 mg, about 550 mg, about 560 mg, about 570 mg, about 580 mg, about 590 mg, about 600 mg, about 610 mg, about 620 mg, about 630 mg, about 640 mg, about 650 mg, about 660 mg, about 670 mg, about 680 mg, about 690 mg, about 700 mg, about 710 mg, about 720 mg, about 730 mg, about 740 mg, about 750 mg, about 760 mg, about 770 mg, about 780 mg, about 790 mg, about 800 mg, about 810 mg, about 820 mg, about 830 mg, about 840 mg, about 850 mg, about 860 mg, about 870 mg, about 880 mg, about 890 mg, about 900 mg, about 910 mg, about 920 mg, about 930 mg, about 940 mg, about 950 mg, about 960 mg, about 970 mg, about 980 mg, about 990 mg, about 1000 mg, about 1010 mg, about 1020 mg, about 1030 mg, about 1040 mg, about 1050 mg, about 1060 mg, about 1070 mg, about 1080 mg, about 1090 mg, about 1100 mg, about 1110 mg, about 1120 mg, about 1130 mg, about 1140 mg, about 1150 mg, about 1160 mg, about 1170 mg, about 1180 mg, about 1190 mg, about 1200 mg, about 1210 mg, about 1220 mg, about 1230 mg, about 1240 mg, about 1250 mg, about 1260 mg, about 1270 mg, about 1280 mg, about 1290 mg, about 1300 mg, about 1310 mg, about 1320 mg, about 1330 mg, about 1340 mg, about 1350 mg, about 1360 mg, about 1370 mg, about 1380 mg, about 1390 mg, about 1400 mg, about 1410 mg, about 1420 mg, about 1430 mg, about 1440 mg, about 1450 mg, about 1460 mg, about 1470 mg, about 1480 mg, about 1490 mg, about 1500 mg, about 1510 mg, about 1520 mg, about 1530 mg, about 1540 mg, about 1550 mg, about 1560 mg, about 1570 mg, about 1580 mg, about 1590 mg, about 1600 mg, about 1610 mg, 1620 mg, about 1630 mg, about 1640 mg, about 1650 mg, about 1660 mg, about 1670 mg, about 1680 mg, about 1690 mg, about 1700 mg, about 1710 mg, about 1720 mg, about 1730 mg, about 1740 mg, about 1750 mg, about 1760 mg, about 1770 mg, about 1780 mg, about 1790 mg, about 1800 mg, about 1810 mg, about 1820 mg, about 1830 mg, about 1840 mg, about 1850 mg, about 1860 mg, about 1870 mg, about 1880 mg, 1890 mg, about 1900 mg, about 1910 mg, about 1920 mg, about 1930 mg, about 1940 mg, about 1950 mg, about 1960 mg, about 1970 mg, about 1980 mg, about 1990 mg, or about 2000 mg.

In a preferred embodiment, the bispecific anti-EGFR/c-Met antibody or pharmaceutical composition thereof is administered at a dose of 400 mg, 800 mg, 1000 mg, 1200 mg, 1400 mg, 1600 mg, 1800 mg, or 2000 mg.

In a preferred embodiment, the above bispecific anti-EGFR/c-Met antibody or pharmaceutical composition thereof is administered at a dose of 400 mg, 800 mg, 1200 mg, 1600 mg, or 2000 mg.

In some embodiments, the bispecific anti-EGFR/c-Met antibody or pharmaceutical composition thereof is administered once a day. In some embodiments, the bispecific anti-EGFR/c-Met antibody or composition thereof is administered once every two days. In some embodiments, the bispecific anti-EGFR/c-Met antibody or composition thereof is administered once every three days. In some embodiments, the bispecific anti-EGFR/c-Met antibody or composition thereof is administered twice a week. In some embodiments, the bispecific anti-EGFR/c-Met antibody or composition thereof is administered once a week. In some embodiments, the bispecific anti-EGFR/c-Met antibody or composition thereof is administered once every two weeks. In some embodiments, the bispecific anti-EGFR/c-Met antibody or composition thereof is administered once every three weeks. In some embodiments, the bispecific anti-EGFR/c-Met antibody or composition thereof is administered once a month.

In a preferred embodiment, the bispecific anti-EGFR/c-Met antibody or pharmaceutical composition thereof is administered at a frequency of once a week for the first treatment cycle, followed by once every two weeks, wherein every four weeks is a treatment cycle.

In a preferred embodiment, the bispecific anti-EGFR/c-Met antibody or pharmaceutical composition thereof is administered at a frequency of once a week for the first treatment cycle, followed by once every three weeks, wherein every three weeks is a treatment cycle.

In a preferred embodiment, the bispecific anti-EGFR/c-Met antibody or pharmaceutical composition thereof is administered at a dose of 400 mg, 800 mg, 1200 mg, 1600 mg, or 2000 mg; and a frequency of once a week for the first treatment cycle, and once every two weeks starting from the second treatment cycle, wherein every four weeks is a treatment cycle. The present invention provides a method of treating or preventing a cancer, comprising administering the bispecific antibody or the bispecific antibody pharmaceutical composition to a subject in need thereof.

The present invention provides a method of treating or preventing a cancer, comprising a combined administration of the bispecific antibody or the bispecific antibody pharmaceutical composition and the EGFR inhibitor to a subject in need thereof, wherein the combined administration may be a simultaneous, concurrent, sequential, continuous, alternating, or separate administration.

In some embodiments, the cancer is a solid tumor; preferably an epithelial cell carcinoma, breast cancer, ovarian cancer, lung cancer, rectal cancer, anal cancer, prostate cancer, kidney cancer, bladder cancer, head and neck cancer, pharyngeal cancer, nasal cancer, pancreatic cancer, skin cancer, oral cancer, tongue cancer, esophageal cancer, vaginal cancer, endometrial cancer, cervical cancer, spleen cancer, testicular cancer, gastric cancer, thymic cancer, colon cancer, thyroid cancer, liver cancer or melanoma, more preferably a non-small cell lung cancer, a lung adenocarcinoma, a lung squamous cell cancer, a large cell lung cancer, a small cell lung carcinoma, a pulmonary sarcomatoid carcinoma, or hepatocellular carcinoma.

In a preferred embodiment, the cancer is a locally advanced or metastatic non-small cell lung cancer. Preferably, the cancer is a locally advanced or metastatic non-small cell lung cancer with EGFR mutations.

In some embodiments, the EGFR mutations comprise at least one mutation selected from the group consisting of L858R mutation, T790M mutation, L861Q mutation, C797S mutation, one or more insertion mutations in EGFR exon 20, and deletion mutation(s) of one or more amino acids in EGFR exon 19.

In a preferred embodiment, the cancer is non-small cell lung cancer with deletion mutation(s) of one or more amino acids in EGFR exon 19, or L858R mutation.

In vitro pharmacodynamic test results indicate that the bispecific anti-EGFRxc-Met antibody of the present invention or the composition thereof is able to bind to cynomolgus monkey EGFR and c-MET, and enhanced ADCC activity can be detected. In vivo pharmacodynamic test results show that the tumor-suppressive effect of the bispecific anti-EGFRxc-Met antibody of the present invention was superior to that of Amivantamab, a marketed similar drug. The tumor-suppressive effect of the bispecific anti-EGFRxc-Met antibody of the present invention, combined with osimertinib, is significantly stronger than that of either single drug, demonstrating good synergistic effects; with no new toxicity observed in any animal in the co-administration group. All animals in the tumor models with EGFR triple mutation and EGFR-20 exon insertion mutation tolerated the treatment well, with good inhibition of tumor growth. It exhibits bioactivity and therapeutic potential for the treatment of advanced malignancies.

In some embodiments, the bispecific antibody comprises a first antibody or antigen-binding fragment against the first target c-Met, comprising a heavy chain variable region as set forth in SEQ ID NO: 07or having at least 90%, 91%, 92%, 93%, 94%, 95%, 96%, 97%, 98% or 99% identity to SEQ ID NO: 07, and a light chain variable region as set forth in SEQ ID NO: 08 or having at least 90%, 91%, 92%, 93%, 94%, 95%, 96%, 97%, 98% or 99% identity to SEQ ID NO: 08; and, a second antibody or antigen-binding fragment against the second target EGFR, comprising a heavy chain variable region as set forth in SEQ ID NO: 15 or having at least 90%, 91%, 92%, 93%, 94%, 95%, 96%, 97%, 98%, or 99% identity to SEQ ID NO: 15, and a light chain variable region as set forth in SEQ ID NO: 16 or having at least 90%, 91%, 92%, 93%, 94%, 95%, 96%, 97%, 98%, or 99% identity to SEQ ID NO: 16.

In some embodiments, the bispecific antibody comprises a first antibody or antigen-binding fragment against the first target c-Met, comprising a heavy chain as set forth in SEQ ID NO: 21 or having at least 90%, 91%, 92%, 93%, 94%, 95%, 96%, 97%, 98% or 99% identity to SEQ ID NO: 21, and a light chain as set forth in SEQ ID NO: 22 or having at least 90%, 91%, 92%, 93%, 94%, 95%, 96%, 97%, 98% or 99% identity to SEQ ID NO: 22; and a second antibody or antigen-binding fragment against the second target EGFR, comprising a heavy chain as set forth in SEQ ID NO: 23 or having at least 90%, 91%, 92%, 93%, 94%, 95%, 96%, 97%, 98%, or 99% identity to SEQ ID NO: 23, and a light chain as set forth in SEQ ID NO: 24, or a light chain having at least 90%, 91%, 92%, 93%, 94%, 95%, 96%, 97%, 98%, or 99% identity to SEQ ID NO: 24.

In some embodiments, the EGFR activating mutation is T790M/L858R, D770-N771 ins_SVD, Del19/T790M/C797S, or exon 19 deletion.

In some embodiments, the bispecific antibody pharmaceutical composition has a unit concentration of 1 mg/mL, 10 mg/mL, 15 mg/mL, 20 mg/mL, 25 mg/mL, 30 mg/mL, 35 mg/mL, 40 mg/mL, 45 mg/mL, or 50 mg/mL.

In some embodiments, the bispecific antibody pharmaceutical composition further comprises a buffer and sucrose.

In some embodiments, the buffer is citric acid-sodium citrate at a concentration of 5 to 150 mM, for example 10 mM; and the concentration of sucrose is 20 to 60 mg/mL, for example 40 mg/mL.

In some embodiments, if the first dose of the bispecific anti-EGFR/c-Met antibody or pharmaceutical composition thereof is more than 400 mg, it is administered on the first and second days of the first cycle, with 400 mg administered on the first day, and the remainder of the dose administered on the second day; no divided administration is required for the second and subsequent doses.

The present invention provides a bispecific antibody pharmaceutical composition, wherein the bispecific antibody pharmaceutical composition is the bispecific antibody pharmaceutical composition described above.

In a preferred embodiment, the bispecific antibody pharmaceutical composition further comprises an EGFR inhibitor selected from one or more of almonertinib, erlotinib, gefitinib, befotertinib, osimertinib, furmonertinib, oritinib, Rezetinib, Avitinib, olmutinib, and rociletinib; and preferably is almonertinib or osimertinib.

The present invention provides a bispecific antibody pharmaceutical composition for use in treating or preventing a cancer, wherein the bispecific antibody pharmaceutical composition is the bispecific antibody pharmaceutical composition described above.

In a preferred embodiment, the bispecific antibody pharmaceutical composition further comprises an EGFR inhibitor selected from one or more of almonertinib, erlotinib, gefitinib, befotertinib, osimertinib, furmonertinib, oritinib, Rezetinib, Avitinib, olmutinib, and rociletinib; and preferably is almonertinib or osimertinib.

### Description of the Drawings

FIG. 1 shows the quality identification results of the bispecific antibody EGFRxc-Met-1. Panel A shows the purity of the bispecific antibody obtained by affinity purification, and panel B shows the proportion of correctly paired products of the purified bispecific antibody.
FIG. 2 shows the effect of the combination of bispecific antibody EGFRxc-Met-1 and almonertinib on tumor growth of subcutaneous xenograft tumors in PC-9 nude mice.

### Detailed Description of the Invention

### Terminology

For easier understanding of the present application, certain technical and scientific terms are specifically defined below. All other technical and scientific terms used herein have the meanings commonly understood by one of ordinary skill in the art to which this application pertains, unless clearly defined elsewhere in this document.

When a list is provided, it is to be understood that each individual element in the list and each combination of the list is a separate embodiment unless otherwise indicated. For example, a list of embodiments presented as "A, B, or C" will be understood to include embodiments "A", "B", "C", "A or B", "A or C", "B or C", or "A, B, or C".

"About" refers to being within an acceptable error range of a particular value as determined by one of ordinary skill in the art, which will depend in part on how the value is measured or determined, i.e., the limitations of the measurement system. In the context of a particular assay, result, or embodiment, "about" means within one standard deviation according to practice in the art or a range of up to 5%, whichever is greater, unless otherwise explicitly stated in the Examples or elsewhere in the specification.

The three-letter codes and one-letter codes for amino acids used herein are described in J. Biol. Chem, 243, p3558(1968).

As used herein, the term "antibody" refers to an immunoglobulin, which is a tetrapeptide chain structure formed by two identical heavy chains and two identical light chains linked by interchain disulfide bonds. The amino acid composition and arrangement order of the constant regions of immunoglobulin heavy chains differ, and therefore their antigenicity differs. Accordingly, immunoglobulins can be divided into five categories, or called isotypes of immunoglobulins, namely IgM, IgD, IgG, IgA, and IgE, and their corresponding heavy chains are µ chain, δ chain, γ chain, α chain, and ε chain, respectively. The same type of Ig can be divided into different subcategories according to the differences in the amino acid composition of its hinge region and the number and position of heavy chain disulfide bonds. For example, IgG can be divided into IgG1, IgG2, IgG3, and IgG4. Light chains are divided into kappa chains or lambda chains based on differences in constant regions. Each of the five categories of Ig can be either a kappa chain or a lambda chain.

In the present application, the antibody light chain variable region described herein may further comprise a light chain constant region comprising a human-derived or murine-derived kappa, lambda chain, or a variant thereof.

In the present application, the antibody heavy chain variable region described herein may further comprise a heavy chain constant region comprising human-derived or murine-derived IgG1, IgG2, IgG3, IgG4, or a variant thereof.

The sequences of about 110 amino acids near the N-terminus of the antibody heavy chain and light chain vary greatly, and constitute a variable region (V region). The sequence of remaining amino acids near the C-terminus is relatively stable and constitute a constant region (C region). The variable region includes three hypervariable regions (HVRs) and four framework regions (FRs) whose sequences are relatively conserved. Three hypervariable regions, also known as complementarity-determining regions (CDRs), determine the specificity of antibodies. Each light chain variable region (VL) or heavy chain variable region (VH) consists of three CDR regions and four FR regions, and the order from amino terminus to carboxyl terminus is: FR1, CDR1, FR2, CDR2, FR3, CDR3, FR4. The three CDR regions of the light chain refer to LCDR1, LCDR2, and LCDR3. The three CDR regions of the heavy chain refer to HCDR1, HCDR2, and HCDR3. The number and position of the CDR amino acid residues in the VL and VH regions of the antibodies or antigen-binding fragments described herein are determined according to known Kabat numbering rules and Kabat or ABM definition rules (http://bioinf.org.uk/abs/).

The term "EGFR" as used herein refers to human epidermal growth factor receptor 1, also known as ErbB-1 or HER1. EGFR is a receptor tyrosine kinase comprising an extracellular ligand-binding domain, a membrane-spanning domain, and an intracellular kinase domain. Upon binding to its ligands, such as epidermal growth factor (EGF) and transforming growth factor α (TGFα), EGFR forms homodimers or heterodimers with other ErbB receptors, and its kinase function is activated, leading to autophosphorylation of several tyrosines in the intracellular domain. An anti-EGFR antibody is an antibody that specifically binds to EGFR. In particular embodiments, the anti-EGFR antibody can interfere with or inhibit EGFR activation, for example, by preventing ligand binding and/or receptor dimerization. After activation, EGFR can transmit signals to downstream effectors, including PI3-K, RAS-RAF-MAPK P44/P42 and protein kinase C signaling pathway, and finally transmit signals to the nucleus to regulate cell proliferation.

As used herein, the term "EGFR exon 20 insertion mutation", or "EGFR exon 20 mutation", or "EGFR Exon 20ins" or "Exon 20ins" refers to a mutation of insertion or duplication of at least one base that occurs within amino acids 762 to 823 of human EGFR. EGFR exon 20 insertion mutations can be detected using known methods, such as sequencing, including next-generation sequencing (NGS), fluorescence in situ hybridization, immunohistochemistry, flow cytometry, or Western blotting.

"Cancer expressing EGFR and/or c-Met" refers to a cancer having detectable expression of EGFR and/or c-Met or having mutations or amplifications of EGFR and/or c-Met. EGFR or c-Met expression, amplification, and mutation status can be detected using known methods, such as sequencing, including next-generation sequencing, fluorescence in situ hybridization, immunohistochemistry, flow cytometry, or Western blotting.

The term "recombinant human antibody" encompasses human antibodies prepared, expressed, created or isolated by recombinant methods involving techniques and methods well known in the art, such as:
1. Antibodies isolated from transgenic, transchromosomal animals (e.g., mice) of human immunoglobulin genes or hybridomas prepared therefrom;
2. Antibodies isolated from a host cell, such as a transfectoma, which has been transformed to express the antibody;
3. An antibody isolated from a recombinant combinatorial human antibody library; and
4. Antibodies prepared, expressed, created, or isolated by methods such as splicing human immunoglobulin gene sequences to other DNA sequences.

Such recombinant human antibodies comprise variable and constant regions that utilize specific human germline immunoglobulin sequences encoded by germline genes, but also include subsequent rearrangements and mutations, such as during antibody maturation.

The term "humanized antibody", also referred to as a CDR-grafted antibody, refers to an antibody produced by grafting the CDR sequences of a mouse into the frameworks of antibody variable regions of a human. Humanized antibodies can overcome the disadvantages of strong immune responses induced by chimeric antibodies because they carry a large number of mouse protein components. In order to avoid a decrease in activity along with a decrease in immunogenicity, the variable region of the human antibody may be subjected to minimal reverse mutations to maintain activity.

The term "antigen-binding fragment" refers to antigen-binding fragments and antibody analogs of antibodies, which typically include at least part of the antigen-binding or variable regions (e.g., one or more CDRs) of the parental antibody. The antibody fragment retains at least some of the binding specificity of the parent antibody. Typically, an antibody fragment retains at least 10% of the parent binding activity when the activity is expressed on a molar basis. Preferably, the antibody fragment retains at least 20%, 50%, 70%, 80%, 90%, 95% or 100% or more of the binding affinity of the parent antibody for the target. Examples of antigen-binding fragments include, but are not limited to: Fab, Fab', F (ab')2, Fv fragments, linear antibodies, single-chain antibodies, nanobodies, domain antibodies, and multispecific antibodies. Engineered antibody variants are reviewed in Holliger and Hudson, 2005, Nat. Biotechnol. 23: 1126-11.

The "Fab fragment" consists of one light chain and the CH1 and the variable region of one heavy chain. The heavy chain of a Fab molecule cannot form a disulfide bond with another heavy chain molecule.

The "Fc" region contains two heavy chain fragments comprising the CH1 and CH2 domains of the antibody. The two heavy chain fragments are held together by two or more disulfide bonds and by the hydrophobic interaction of the CH3 domain.

The "Fab' fragment" contains one light chain, and a portion of one heavy chain comprising a VH domain and a CH1 domain and a region between the CH1 and CH2 domains, whereby interchain disulfide bonds can be formed between the two heavy chains of the two Fab' fragments to form an F (ab')2 molecule.

The "F(ab')2 fragment" contains two light chains and two heavy chains comprising portions of the constant region between the CH1 and CH2 domains, thereby forming an interchain disulfide bond between the two heavy chains. Thus, the F(ab')2 fragment consists of two Fab' fragments held together by disulfide bonds between the two heavy chains.

The "Fv region" comprises a variable region from both heavy and light chains, but lacks constant regions.

The term "multispecific antibody" is used in its broadest sense and encompasses antibodies having multiple epitope specificities. These multispecific antibodies include, but are not limited to: An antibody comprising a heavy chain variable region VH and a light chain variable region VL, wherein the VH-VL unit has multi-epitope specificity; An antibody having two or more VL and VH regions, each VH-VL unit binding to a different target or a different epitope on a same target; An antibody having two or more single variable regions, each single variable region binding to a different target or a different epitope on a same target; a Full-length antibody, an antibody fragment, a diabodies, a bispecific diabodies, and a triabodies, antibody fragments that have been covalently or non-covalently linked together, and the like.

The term "single-chain antibody" is a single-chain recombinant protein formed by linking the heavy chain variable region VH and the light chain variable region VL of an antibody by a peptide linker, which is the smallest antibody fragment having a complete antigen binding site.

The term "domain antibody fragment" is an immunoglobulin fragment having immunological function, containing only a heavy chain variable region or a light chain variable region chain. In certain instances, two or more VH regions are covalently linked to a peptide linker to form a bivalent domain antibody fragment. The two VH regions of the bivalent domain antibody fragment can target a same antigen or different antigens.

The term "single-arm antibody" as used herein refers to an antigen-binding fragment, comprising a Fab fragment, typically comprising a heavy chain (e.g., VH and CH1) and a light chain (e.g., VL and CL), and an Fc fragment, comprising a constant region (e.g., CH2 and CH3). The Fab segment and the Fc segment may be connected by a linker or not by a linker. The single-arm antibody of the present invention can be prepared or synthesized by various methods, for example, constructing a sequence encoding a Fab heavy chain and a sequence encoding an Fc into the same vector, constructing a sequence encoding a Fab light chain into another vector, and transforming the two vectors into a host cell, respectively, to obtain a single-arm antibody.

The term "antigen-binding site" herein refers to a three-dimensional spatial site recognized by an antibody or antigen-binding fragment of the present application.

The term "epitope" refers to a site on an antigen that specifically binds to an immunoglobulin or antibody. An epitope may be formed by adjacent amino acids or non-adjacent amino acids brought into proximity by tertiary folding of a protein. Epitopes formed by adjacent amino acids are typically maintained after exposure to denaturing solvents, while epitopes formed by tertiary folding are typically lost after treatment with denaturing solvents. Epitopes typically comprise at least 3-15 amino acids in a unique spatial conformation. Methods for determining what epitope is bound by a given antibody are well known in the art, including immunoblotting and immunoprecipitation detection assays, among others. Methods for determining the spatial conformation of an epitope include techniques in the art and those described herein, such as X-ray crystal analysis and two-dimensional nuclear magnetic resonance, among others.

As used herein, the terms "specifically bind to" and "selectively bind to" refer to the binding of an antibody to an epitope on a predetermined antigen. Typically, when determined in an instrument by surface plasmon resonance (SPR) technique using human EGFR or c-Met as an analyte and using an antibody as a ligand, an antibody binds to a predetermined antigen with an equilibrium dissociation constant (KD) of approximately less than 10⁻⁷ M or even less, and binds to the predetermined antigen with an affinity that is at least twice that it binds to a non-specific antigen other than the predetermined antigen or a closely related antigen, such as BSA and the like. The term "antibody that recognizes an antigen" is used interchangeably herein with the term "antibody that specifically binds to".

The terms "inhibition" or "blocking" are used interchangeably and encompass both partial and complete inhibition/blocking. Inhibition/blocking of the ligand preferably reduces or alters the normal level or type of activity that occurs when ligand binding occurs without the inhibition or blocking. Inhibition and blocking are also intended to include any measurable reduction in ligand binding affinity when contacted with the antibody, as compared to the ligand not contacted with the antibody.

The term "inhibiting growth" (e.g., relating to cells) is intended to include any measurable reduction in cell growth.

The terms "inducing an immune response" and "enhancing an immune response" are used interchangeably and refer to stimulation (i.e., passive or adaptive) of an immune response to a particular antigen. The term "induction" with respect to the induction of CDC or ADCC refers to the stimulation of a specific direct cell killing mechanism.

As used herein, "ADCC", that is, antibody-dependent cell-mediated cytotoxicity, refers to a cell expressing an Fc receptor that directly kills a target cell coated with an antibody by recognizing the Fc fragment of the antibody. The ADCC effector function of the antibody can be enhanced, decreased, or eliminated by modification of the Fc fragment on the IgG. The modification refers to a mutation in the heavy chain constant region of an antibody.

Methods for producing and purifying antibodies and antigen-binding fragments are well known and can be found in the prior art, such as Antibodies: A Laboratory Manual, Cold Spring Harbor Laboratory Press, Chapters 5-8 and 15. For example, a mouse can be immunized with human BCMA or a fragment thereof, and the resulting antibodies can be renatured, purified, and amino acid sequencing can be performed by conventional methods. Antigen-binding fragments can likewise be prepared by conventional methods. The antibody or antigen-binding fragment of the present invention is genetically engineered to add one or more human FR regions to CDR regions of non-human origin. Human FR germline sequences can be obtained from ImMunoGeneTics (IMGT) website at http://imgt.cines.fr, or from the Journal of Immunoglobulin, 2001, ISBN 012441351.

The engineered antibodies or antigen-binding fragments of the present application can be prepared and purified by conventional methods. The cDNA sequences of the corresponding antibodies can be cloned and recombined into a GS expression vector. The recombinant immunoglobulin expression vector can be stably transfected into CHO cells. As a more recommended prior art, mammalian expression systems lead to glycosylation of antibodies, particularly at the highly conserved N-terminus of the FC region. Stable clones may be obtained by expressing antibodies that specifically bind to human antigens. Antibodies may be produced by expansion of a positive clone in serum-free medium in a bioreactor. The culture medium with secreted antibodies can be purified and collected by conventional techniques. The antibodies can be concentrated by filtration using conventional methods. Soluble mixtures and multimers can also be removed by conventional methods, such as molecular sieves and ion exchange. The resulting product needs to be immediately frozen, e.g., at-70°C, or lyophilized.

The term "pharmaceutical composition" refers to a product comprising one or more active ingredients (e.g., antibodies, small molecule drugs) in optionally specified amounts, as well as any product directly or indirectly produced by combining one or more active ingredients in optionally specified amounts. The different active ingredients in the pharmaceutical composition can be administered independently in separate formulations, either simultaneously or at different time points, to achieve a combined synergistic effect. In the present disclosure, "pharmaceutical composition" and "formulation" are not incompatible with each other.

The term "combined" or "combination" refers to a mode of administration that refers to the administration of at least one dose of a bispecific antibody or bispecific antibody pharmaceutical composition, and at least one dose of an additional therapeutic agent over a period of time, wherein both substances exhibit a pharmacological effect. The time period may be within a dosing cycle, preferably within 4 weeks, within 3 weeks, within 2 weeks, within 1 week, or within 24 hours, more preferably within 12 hours. The bispecific antibody or bispecific antibody pharmaceutical composition and the additional therapeutic agent may be administered simultaneously or sequentially. Such periods include treatments in which the bispecific antibody or bispecific antibody pharmaceutical composition and the additional therapeutic agent are administered by the same route of administration or different routes of administration. The mode of administration of the combination described herein is selected from the group consisting of simultaneous administration, independent formulation and co-administration, or independent formulation and sequential administration.

The terms "administration", "giving", and "treatment", when applied to an animal, human, experimental subject, cell, tissue, organ, or biological fluid, refer to the contact of an exogenous drug, therapeutic agent, diagnostic agent, or composition with the animal, human, subject, cell, tissue, organ, or biological fluid. "Administration", "giving", and "treatment" can refer to, for example, therapeutic, pharmacokinetic, diagnostic, research, and experimental methods. The treatment of the cells includes contacting the reagent with the cells, and contacting the reagent with a fluid, wherein the fluid is in contact with the cells. "Administration", "giving", and "treatment" also mean treating, for example, cells in vitro and ex vivo by an agent, a diagnostic, a binding composition, or by another cell. "Treatment", when applied to human, veterinary, or research subjects, refers to therapeutic treatment, prophylactic or preventive measures, research, and diagnostic applications.

The term "treatment" means administering an internal or topical therapeutic agent, such as comprising any one of the antibodies of the present application, to a patient having one or more symptoms of a disease for which the therapeutic agent is known to have a therapeutic effect. Typically, the therapeutic agent is administered to the patient or population being treated in an amount effective to alleviate one or more symptoms of the disease, whether by inducing regression of such symptoms or inhibiting the progression of such symptoms to any clinically measurable extent. The amount of a therapeutic agent effective to alleviate the symptoms of any particular disease (also referred to as a "therapeutically effective amount") may vary depending on a variety of factors, such as the state of disease, the age and weight of the patient, and the ability of the drug to produce the desired therapeutic effect in the patient. Whether a disease symptom has been alleviated can be assessed by any clinical test commonly used by a physician or other health care professional to assess the severity or progression of the symptom. Embodiments of the present application (e.g., methods of treatment, or articles of manufacture) may be ineffective in alleviating the symptoms of the target disease present in each patient, but should alleviate the symptoms of the target disease in a statistically significant number of patients as determined by any statistical test known in the art, such as Student's t-test, chi-square test, U-test according to Mann and Whitney, Kruskal-Wallis test (H-test), Jonckheere-Terpstra test, and Wilcoxon test.

The term "preventing" a disease or disorder means preventing the occurrence of the disorder in a subject.

The term "diagnosis" refers to a method of determining whether a subject suffers from a given disease or disorder, whether the subject may develop a given disease or disorder in the future, or whether the subject is likely to respond to treatment for a previously diagnosed disease or disorder (i.e., stratifying a patient population according to likelihood of responding to a treatment). Diagnosis is typically performed by a physician based on general guidelines for the disease to be diagnosed or other criteria indicating that the subject is likely to respond to a particular treatment.

The term "consisting essentially of" or variations thereof, as used throughout the specification and claims, indicates the inclusion of all said elements or groups of elements, and optionally other elements of similar or different properties to said elements, which do not significantly alter the essential or new properties of the specified dosing regimen, method, or composition.

As used herein, the term "naturally occurring" as applied to an object refers to the fact that the object can be found in nature. Polypeptide sequences or polynucleotide sequences are naturally occurring if they are, for example, in organisms (including viruses) that can be isolated from sources in nature and that have not been intentionally modified artificially in the laboratory.

The term "effective amount" includes an amount sufficient to ameliorate or prevent a symptom or condition of a medical disorder. An effective amount also means an amount sufficient to allow or facilitate diagnosis. The effective amount for a particular patient or veterinary subject may vary depending on factors such as the condition to be treated, the general health of the patient, the method, route, and dosage of administration, and the severity of side effects. An effective amount may be the maximum dose or dosing regimen that avoids significant side effects or toxic effects.

The term "exogenous" refers to that a substance is produced outside an organism, cell, or human body, depending on the context.

The term "endogenous" refers to that a substance is produced in a cell, organism, or human body, depending on the context.

The term "homology" refers to sequence similarity between two polynucleotide sequences or between two polypeptides. When a position in two compared sequences is occupied by the same base or amino acid monomer subunit, for example, if each position of two DNA molecules is occupied by adenine, then the molecules are homologous at that position. The percentage of homology between two sequences is a function of the number of matched or homologous positions shared by the two sequences divided by the number of compared positions × 100%. For example, when the sequences are optimally aligned, two sequences are 60% homologous if there are 6 matches or homologous at 10 positions in the two sequences. In general, comparisons are made when two sequences are aligned to obtain the greatest percentage of homology.

As used herein, the expressions "cell", "cell line", and "cell culture" are used interchangeably, and all such designations include their progeny. Thus, the words "transformant" and "transformed cell" include the primary test cell and the culture derived therefrom, regardless of the number of transformed cells. It should also be understood that all progeny cannot be precisely identical in terms of DNA content due to deliberate or undeliberate mutations. Mutant progeny having the same function or biological activity as that screened for in the initially transformed cells are included. Where different names are referred to, this will be clear from the context.

"Optional" or "optionally" means that the event or condition described subsequently may, but need not, occur, and the description includes instances where the event or environment occurs or does not occur. For example, "optionally comprising 1-3 antibody heavy chain variable regions" means that antibody heavy chain variable regions of a particular sequence may, but need not, be present.

### Example

The following examples are used to further describe the application, but these examples are not intended to limit the scope of the application. Experimental methods in the embodiments of the present application that do not specify particular conditions are generally carried out under conventional conditions, such as Antibodies: A Laboratory Manual, and Molecular Cloning: A Laboratory Manual, Cold Spring Harbor Laboratory Press; or under the conditions recommended by the raw material or product manufacturers. Reagents without indicated specific sources are conventional and commercially available.

### Example 1 Construction and Preparation of Bispecific Antibodies

### Screening of monoclonal antibodies

The sequence of anti-c-MET antibody-1 used in the present application was obtained by immunizing mice with the antigen mesenchymal-epithelial transition factor (c-MET) (purchased from AcroBiosystems), extracting total RNA, performing reverse transcription, and constructing a yeast display library by PCR amplification for screening. The sequence of antibody-1 was obtained by sequencing, wherein the CDR sequences of the antibody were identified by the IMGT numbering system (Lefranc et al., Dev. Comparat. Immunol. 27:55-77, 2003).

Specifically, a fully human-derived anti-c-MET antibody library was first constructed from five immunized mice with a diversity of up to 3 x 107, and human c-MET protein was labeled according to the product instructions of the biotin labeling kit (purchased from Thermo). Yeast cells that can specifically bind to c-MET were enriched by MACS, and after several rounds of FACS-sorting, yeast cells that specifically bind to biotin-labeled c-MET protein with relatively high affinity were finally obtained. Using the finally sorted yeast as a template, the sequences of the antibody heavy and light chains were retrieved and constructed into expression vectors to prepare the antibody.

**Table 1. Sequence information of cMET antibody-1**

| cMET antibody-1 | | | | | |
|---|---|---|---|---|---|
| HCDR1 | HCDR2 | HCDR3 | LCDR1 | LCDR2 | LCDR3 |
| SEQ ID NO: 01 | SEQ ID NO: 02 | SEQ ID NO: 03 | SEQ ID NO: 04 | SEQ ID NO: 05 | SEQ ID NO: 06 |
| GGSVTSVNYY | ISYSGNT | VRAPYYYMDV | QGISSW | AVS | QQANSFPLT |
| Heavy chain variable region VH (SEQ ID NO: 07) | | | Light chain variable region VL (SEQ ID NO: 08) | | |
| EVQLQESGPGLVKPSETLSLTCTVSGGSVTSVNY YWKWIRQPPGKGLEWIGYISYSGNTNYNPSLKS RVTISVDTSKNQFSLKLSSVTAADTAVYYCVRAP YYYMDVWSKGTTVTVSS | | | DIVMTQSPSSVSASVGDRVTITCRASQGISS WLAWYQQKPGKAPKLLIYAVSSLQSGVPS RFSGSGSGTDFTLTISSLQPEDFATYFCQQA NSFPLTFGGGTKVEIK | | |

After antibody preparation, ForteBio affinity measurements were performed according to the reported method (Estep, P et al., High throughput solution-based measurement of antibody-antigen affinity and epitope binning. MAbs, 2013.5 (2): p.270-8). The results showed that anti-c-MET antibody-1 had good binding activity to human c-MET protein and monkey c-MET protein.

**Table 2. Affinity of candidate molecules to human c-Met**

| Number | KD(M) | Kon(1/Ms) | Koff(1/s) |
|---|---|---|---|
| c-MET antibody-1 | 2.93E-11 | 2.29E+05 | 6.71E-06 |

**Table 3. Affinity of candidate molecules to monkey c-Met**

| Number | KD(M) | Kon(1/Ms) | Koff(1/s) |
|---|---|---|---|
| c-MET antibody-1 | 1.16E-09 | 3.69E+05 | 4.26E-04 |

### Construction of anti-cMet and EGFR bispecific antibody

According to the method described in the patent (Patent Application No.: CN201611016435.0), nucleotide sequences encoding the variable regions of anti-EGFR and anti-cMet antibodies were synthesized and linked to constant regions that can spontaneously form a heterodimer. Among them, the anti-cMet antibody is c-MET antibody-1 obtained by the above screening; and the heavy chain variable region and the light chain variable region of the anti-EGFR antibody are disclosed in Patent No.: WO02/100348, wherein the first amino acid Q of the heavy chain variable region was mutated to E, and the first amino acid A of the light chain variable region was mutated to D, and the sequences thereof are specifically shown in Table 4.

**Table 4. Sequence information of the EGFR antibody**

| EGFR antibody | | | | | |
|---|---|---|---|---|---|
| HCDR1 | HCDR2 | HCDR3 | LCDR1 | LCDR2 | LCDR3 |
| SEQ ID NO: 09 | SEQ ID NO: 10 | SEQ ID NO: 11 | SEQ ID NO: 12 | SEQ ID NO: 13 | SEQ ID NO: 14 |
| GFTFSTYG | IWDDGSYK | ARDGITMVRGV MKDYFDY | QDISSA | DAS | QQFNSYPLT |
| Heavy chain variable region VH (SEQ ID NO: 15) | | | Light chain variable region VL (SEQ ID NO: 16) | | |
| EVQLVESGGGVVQPGRSLRLSCAASGFTFSTYGMH WVRQAPGKGLEWVAVIWDDGSYKYYGDSVKGRF TISRDNSKNTLYLQMNSLRAEDTAVYYCARDGITM VRGVMKDYFDYWGQGTLVTVSS | | | DIQLTQSPSSLSASVGDRVTITCRASQDISSA LVWYQQKPGKAPKLLIYDASSLESGVPSRF SGSESGTDFTLTISSLQPEDFATYYCQQFNS YPLTFGGGTKVEIK | | |

The EGFRxcMET-1 molecule was constructed using CH1/CL preference mutation (patent number: WO2021/067404A2) and Knob in Hole technology. The heavy chain variable regions of the anti-cMet antibody and the anti-EGFR antibody were constructed into the CH1 mutant heavy chain constant regions CH SET1 (SEQ ID NO: 17) and CH SET2 (SEQ ID NO: 18), respectively. The light chain variable regions of the anti-cMet antibody and the anti-EGFR antibody were constructed into the CL mutant light chain constant regions CL SET1 (SEQ ID NO: 19) and CL SET2 (SEQ ID NO: 20), respectively. A 1 +1 asymmetric anti-EGFRxcMet bispecific antibody cell line was constructed.

**Table 5. Sequence information of EGFRxcMet bispecific antibody**

| | | |
|---|---|---|
| CH SET1 | SEQ ID NO: 17 | ASTKGPSVFPLAPSSKSTSGGTAALGCQVEDYFPEPVTVSWNSGALTSGVH TFPAVLQSSGLYELSSVVTVPSSSLGTQTYICNVNHKPSNTKVDKKVEPKSC DKTHTCPPCPAPELLGGPSVFLFPPKPKDTLMISRTPEVTCVVVDVSHEDPE VKFNWYVDGVEVHNAKTKPREEQYNSTYRVVSVLTVLHQDWLNGKEYK CKVSNKALPAPIEKTISKAKGQPREPQVYTLPPCRDELTKNQVSLWCLVKGF YPSDIAVEWESNGQPENNYKTTPPVLDSDGSFFLYSKLTVDKSRWQQGNVF SCSVMHEALHNHYTQKSLSLSPG |
| CH SET2 | SEQ ID NO: 18 | ASTKGPSVFPRAPSSKSTSGGTAALGCLVRDYFPEPVTVSWNSGALTSGVHT FPAVLQSSGLYSLSSVVTVPSSSLGTQTYICNVNHKPSNTKVDKKVEPKSCD KTHTCPPCPAPELLGGPSVFLFPPKPKDTLMISRTPEVTCVVVDVSHEDPEV KFNWYVDGVEVHNAKTKPREEQYNSTYRVVSVLTVLHQDWLNGKEYKC KVSNKALPAPIEKTISKAKGQPREPQVCTLPPSRDELTKNQVSLSCAVKGFY PSDIAVEWESNGQPENNYKTTPPVLDSDGSFFLVSKLTVDKSRWQQGNVFS CSVMHEALHNHYTQKSLSLSPG |
| CL SET1 | SEQ ID NO: 19 | RTVAAPSVFIFPPSDEQLKSGRASVVCLLNNFYPREAKVQWKVDNALQSGN SQESVTEQDSKDSTYSLSSRLQLSKADYEKHKVYACEVTHQGLSSPVTKSF NRGEC |
| CL SET2 | SEQ ID NO: 20 | RTVAAPSVFIFPPSDEELKSGTASVQCLLNNFYPREAKVQWKVDNALQSGN |
| | | SQESVTEQDSKDSTYSLSSELTLSKADYEKHKVYACEVTHQGLSSPVTKSFN RGEC |
| cMet HC | SEQ ID NO: 21 | EVQLQESGPGLVKPSETLSLTCTVSGGSVTSVNYYWKWIRQPPGKGLEWIG YISYSGNTNYNPSLKSRVTISVDTSKNQFSLKLSSVTAADTAVYYCVRAPYY YMDVWSKGTTVTVSSASTKGPSVFPLAPSSKSTSGGTAALGCQVEDYFPEP VTVSWNSGALTSGVHTFPAVLQSSGLYELSSVVTVPSSSLGTQTYICNVNHK PSNTKVDKKVEPKSCDKTHTCPPCPAPELLGGPSVFLFPPKPKDTLMISRTPE VTCVVVDVSHEDPEVKFNWYVDGVEVHNAKTKPREEQYNSTYRVVSVLT VLHQDWLNGKEYKCKVSNKALPAPIEKTISKAKGQPREPQVYTLPPCRDEL TKNQVSLWCLVKGFYPSDIAVEWESNGQPENNYKTTPPVLDSDGSFFLYSK LTVDKSRWQQGNVFSCSVMHEALHNHYTQKSLSLSPG |
| cMet LC | SEQ ID NO: 22 | DIVMTQSPSSVSASVGDRVTITCRASQGISSWLAWYQQKPGKAPKLLIYAVS SLQSGVPSRFSGSGSGTDFTLTISSLQPEDFATYFCQQANSFPLTFGGGTKVEI KRTVAAPSVFIFPPSDEQLKSGRASVVCLLNNFYPREAKVQWKVDNALQSG NSQESVTEQDSKDSTYSLSSRLQLSKADYEKHKVYACEVTHQGLSSPVTKS FNRGEC |
| EGFR HC | SEQ ID NO: 23 | EVQLVESGGGVVQPGRSLRLSCAASGFTFSTYGMHWVRQAPGKGLEWVA VIWDDGSYKYYGDSVKGRFTISRDNSKNTLYLQMNSLRAEDTAVYYCARD GITMVRGVMKDYFDYWGQGTLVTVSSASTKGPSVFPRAPSSKSTSGGTAA LGCLVRDYFPEPVTVSWNSGALTSGVHTFPAVLQSSGLYSLSSVVTVPSSSL GTQTYICNVNHKPSNTKVDKKVEPKSCDKTHTCPPCPAPELLGGPSVFLFPP KPKDTLMISRTPEVTCVVVDVSHEDPEVKFNWYVDGVEVHNAKTKPREEQ YNSTYRVVSVLTVLHQDWLNGKEYKCKVSNKALPAPIEKTISKAKGQPREP QVCTLPPSRDELTKNQVSLSCAVKGFYPSDIAVEWESNGQPENNYKTTPPVL DSDGSFFLVSKLTVDKSRWQQGNVFSCSVMHEALHNHYTQKSLSLSPG |
| EGFR LC | SEQ ID NO: 24 | DIQLTQSPSSLSASVGDRVTITCRASQDISSALVWYQQKPGKAPKLLIYDASS LESGVPSRFSGSESGTDFTLTISSLQPEDFATYYCQQFNSYPLTFGGGTKVEIK RTVAAPSVFIFPPSDEELKSGTASVQCLLNNFYPREAKVQWKVDNALQSGN SQESVTEQDSKDSTYSLSSELTLSKADYEKHKVYACEVTHQGLSSPVTKSFN RGEC |

Using the electroporation method, the vector pCHO2.0-GS-Puro-H1-L1 containing the nucleotide sequence encoding the anti-c-Met antibody heavy chain (SEQ ID NO: 21) and the nucleotide sequence encoding the light chain (SEQ ID NO: 22), and the vector pCHO2.0-GS-Puro-H2-L2 containing the nucleotide sequence encoding the anti-EGFR antibody heavy chain (SEQ ID NO: 23) and the nucleotide sequence encoding the light chain (SEQ ID NO: 24), were co-transferred into the host cell CHOS-ADP Fut8 KO (defucosylation gene-knockout cell line). Using selection pressures with Puromycin and MSX, the cells were pressure-seleted to obtain high-yielding minipools. Then, by one round of limited dilution and monoclonal screening, high-yielding and stable clonal cell lines were obtained. After comparing the growth states, protein expression levels, and physical and chemical properties of the expressed proteins, the cloned cell line 6F5 was finally selected as a recombinant engineered cell line.

Dynamis AGT Medium was used as the basal medium. Cells were inoculated at a density of (1.0 ± 0. 2) x 106 cells/mL, and feeds of 7a feed at 5.0 ± 0. 5% (w/w) of the initial culture weight and 7b at 0.5 ± 0. 05% (w/w) of the initial culture weight were added on days 3, 5, 7, 9 and 11, respectively. The dissolved oxygen was set at 40%, the initial culture temperature was 36.5°C, and the temperature was reduced to 33.0°C on the day 4. According to the measurements of glucose concentration, 300 g/kg glucose concentrate was added every day, except on the day of harvest, to reach a glucose concentration of 6.0 g/L in the culture medium. The culture was terminated on day 14 or when the cell viability was less than 80%. During the culture process, cell density and viability were measured using Vicell (Beckman). Antibody production was measured using Cedex (Roche) every day from day 7. As indicated by the results shown in Table 4, the expression level of the bispecific antibody reached 2.432 g/L.

The supernatant was collected and purified using protein A magnetic beads (purchased from GenScript) sorting method to isolate the protein of interest. The magnetic beads were resuspended (1-4 times the volume of the magnetic beads) in an appropriate volume of binding buffer (PBS + 0. 1% Tween 20, pH 7.4), and then added to the sample to be purified, and incubated at room temperature for 1 hour with gentle shaking. The samples were placed on a magnetic rack (purchased from Beaver), the supernatant was discarded, and the magnetic beads were washed 3 times with binding buffer. Elution buffer (0.1 M sodium citrate, pH 3.2) was added at 3-5 times the volume of the magnetic beads, and shaken at room temperature for 5-10 minutes, then the samples were put back on the magnetic rack. The elution buffer was collected and transferred to a collection tube pre-added with neutralization buffer (1 M Tris, pH 8.54), and mixed to obtain the bispecific antibody sample EGFRxcMET-1.

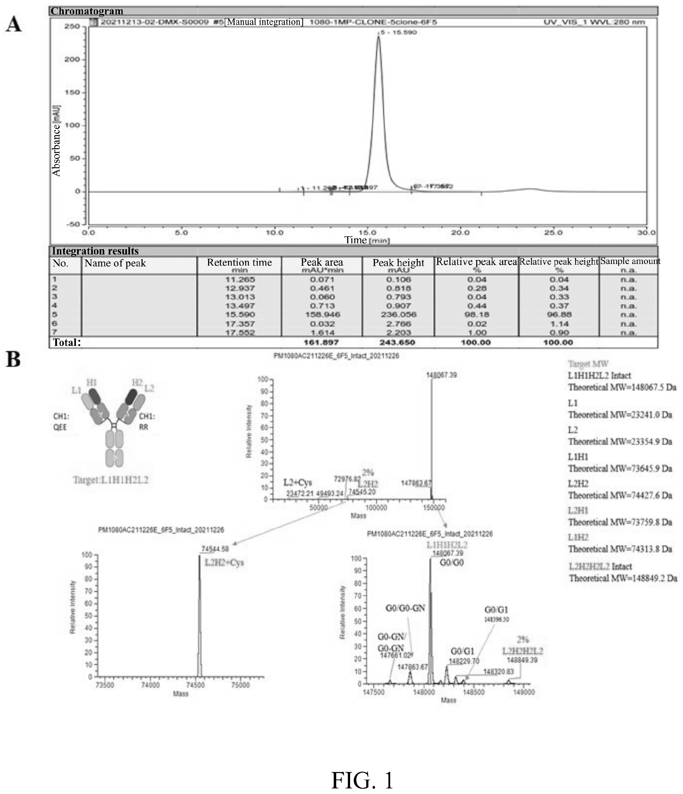

The purity of the obtained protein was determined by HPLC. The HPLC method was as follows: mobile phase: 150 mM Na2HPO4•12H2O, pH 7.0. Chromatographic conditions: detection wavelength 280 nm, column temperature 25 °C, flow rate 0.5 mL/min, detection time: 30 min, TSK gel G3000SWXL chromatographic column. The SEC results, as shown in panel A of FIG. 1, indicated that the purity of the bispecific antibody obtained by one-step affinity purification was 98.18%.

The pairing of heavy and light chains of the protein was determined by high-performance liquid mass spectrometry, with instruments of liquid system VanquishUHPLC (Thermo), mass spectrometer Q Exactive (Thermo), and chromatographic column Waters ACQUITY UPLCBEH C4, 2.1 mm x 10 mm. 50 µg of sample was taken, diluted to 25 µL with ultrapure water, centrifuged, and 20 µL of the sample was transferred to a 10-injection-vial; 5 µL was injected for LC-MS analysis of the intact molecular weight. The chromatographic conditions were: column temperature: 80 °C; UV detection wavelength: 280 nm; flow rate: 0.3 mL/min; mobile phase A: aqueous solution (containing 0.1% formic acid); mobile phase B: acetonitrile solution (containing 0.1% formic acid). The mass spectrometry parameters were: ESI ion source: ion transfer tube temperature 320 °C, voltage 3.8 kV, gas flow rate 36 L/min; mode: positive ion Full MS; resolution: 17500; scan range: 600-4000 m/z. The results, as shown in panel B of FIG. 1, indicated that the proportion of correctly paired products of the purified bispecific antibodies was > 98%.

### Example 2 Preparation of Anti-EGFRxc-Met Bispecific Antibody Injection

Liquid preparations of the bispecific antibody at concentrations of 5, 10, 15, 20, 25, 30, 35, 40, 45, 50 mg/mL were prepared using citric acid-sodium citrate (abbreviation: CA) buffer, optionally with the addition of sucrose.

**Table 6. Components of EGFRxcMet bispecific antibody injection**

| Components | Final concentration |
|---|---|
| EGFRxcMET-1 | 5, 10, 15, 20, 25, 30, 35, 40, 45, 50 mg/mL |
| citric acid-sodium citrate buffer | 10 mM |
| Sucrose | 40 mg/mL |

In order to prepare 1 kg of 10 mM citric acid-sodium citrate buffer, 0.282 g of citric acid monohydrate (C6H8O7·H2O), and 2.547 g of sodium citrate dihydrate (C6H5Na3O7·2H2O) were weighed, added with water to volume to 1 Kg, and then the buffer was filtered. EGFRxcMET-1 was dialyzed into 10 mM citric acid-sodium citrate buffer prepared above by dialysis exchanges, and a sucrose stock solution was added separately to prepare the target formulations with protein concentrations of 5, 10, 15, 20, 25, 30, 35, 40, 45, and 50 mg/mL. In a biosafety cabinet, the formulations were filtered through a 0.22 µm PVDF filter membrane and then packaged into vials, stoppered, and capped to prepare the bispecific antibody injection solution.

### Example 3 Binding Activity of Anti-EGFRxc-Met Bispecific Antibody to EGFR and c-Met Positive Tumor Cells

A375 cells, purchased from Addexbio, are human malignant melanoma cells with low expression of EGFR and c-Met receptors. H292 cells, purchased from Procell, are human lung adenocarcinoma cells with relatively high expression of EGFR and c-Met receptors. HCC827 cells, purchased from Cell Bank for Type Culture Collection, CAS, are human non-small cell lung cancer cells with high expression of EGFR and c-Met receptors. The expanded cultured cells were adjusted to an appropriate cell density, added to a 96-well flow plate, and centrifuged. Then, the gradient-diluted samples to be tested were added and incubated at 4 °C for 1 h. Cells were washed twice with PBS, added with the fluorescent secondary antibody correspondingly diluted to an appropriate concentration, incubated at 4 °C for 30 min, and washed twice with PBS. Cells were resuspended in PBS and tested on a CytoFlex flow cytometer; and the corresponding MFI was calculated.

For tumor cells with different expression levels of EGFR and MET, EGFRxc-Met bispecific antibody candidate molecule EGFRxcMET-1 showed a strong cell binding activity, exhibiting a binding strength with EC50 values as shown in Table 7 below.

**Table 7. Antibody binding activity to tumor cells**

| | A375 EC₅₀(nM) | H292 EC₅₀(nM) | HCC827 EC₅₀(nM) |
|---|---|---|---|
| EGFRxcMET-1 | 1.35 | 2.34 | 3.67 |

### Example 4 Anti-Egfrxc-Met Bispecific Antibody can Induce ADCC Effect

A375 cells, purchased from Addexbio, are human malignant melanoma cells with low expression of EGFR and c-Met receptors. H1975 cells, purchased from Addexbio, are human lung adenocarcinoma cells with relatively high expression of EGFR and c-Met receptors. HCC827 cells, purchased from Cell Bank for Type Culture Collection, CAS, are human non-small cell lung cancer cells with high expression of EGFR and c-Met receptors. The expanded cultured A375/H1975/HCC827 cells were resuspended with 1640 medium at a cell density of 1.2 x 106 cells/mL. CD16a-NFAT-Luc Jurkat reporter cells (in-house-constructed as follows: CD16a and NFAT-Luc gene sequences were constructed on the pCDNA3.1 vector, and then transfected into Jurkat cells. An antibiotic resistance pressure screening was performed to obtain the CD16a-NFAT-Luc Jurkat reporter cells) were resuspended in 1640 medium, with the cell density adjusted to 6 x 106 cells/mL. The antibody was diluted to 300 nM with 1640 medium, then diluted in a 4-fold gradient. 25 µL of the above-mentioned gradient diluted antibody was added to each well of a 96-well cell culture plate with 25 µL of the above-mentioned target cells per well. After mixing, the plate was incubated at room temperature for 30 minutes, then 25 µL of the above-mentioned effector cells was added to each well. After mixing, the plate was incubated at 37°C and 5% CO2 for 6 h, then 75 µL of Bio-turbo reagent (purchased from RHINO BIO) was added to each well, and after mixing, the chemiluminescence signal was detected by a microplate reader.

The EGFRxc-Met bispecific antibody EGFRxMET-1 induced ADCC effect and showed a dependence on the amount of antigen expression (HCC827 > H1975 > A375).

### Example 5 Tumor-Suppressive Effect of Different Doses of Egfrxc-Met Bispecific Antibody in H292 Tumor-Bearing CB-17 SCID Mouse Model

In this study, a tumor-bearing model was established by subcutaneously inoculating CB-17 SCID mice with human lung cancer cells H292 (high expression of EGFR/c-MET), which can be used to evaluate the anti-tumor effect in relation to the mechanism of action of the test article and safety profile under disease conditions. This experiment was divided into 4 groups, each group of six mice, and PBS or 3 different doses (2, 8, 32 mg/kg) of EGFRxcMET-1 were administered intraperitoneally, once every 4 to 6 days, for a total of 3 doses. The suppression effect and safety of different doses of the bispecific antibody on the growth of subcutaneously inoculated human lung cancer cell H292 tumors were investigated in CB-17 SCID mice.

The tumor of mice in the negative control PBS group grew rapidly, and the average tumor volume reached more than 900 mm3 by the end point of the experiment (D30), indicating the successful establishment of the tumor model of the experiment.

At the end of the experiment, tumor volumes in the PBS group and EGFRxcMET-1 groups at 2, 8, and 32 mg/kg were 930.82 mm3, 587.42 mm3(P<0.05), 65.03 mm3(P<0.0001), and 45.35 mm3(P<0.0001), respectively. Compared with the negative control PBS group, each dose group of EGFRxcMET-1 could inhibit tumor growth in a dose-dependent manner, with TGIs of 45%, 113%, and 115%, respectively, and tumor weights of 0.53 g, 0.30 g(P<0.01), 0.03 g(P<0.0001), and 0.01 g(P<0.0001), respectively. The detailed results are shown in Table 8. No abnormalities were observed in the condition of mice of any group. Compared with the control group, the body weight of mice in each treatment group did not decrease significantly. At the end of the experiment, no obvious abnormalities were observed in the main organs during gross necropsy of mice of any group, indicating that the drugs in each group did not produce obvious toxicity to mice under the dosage used in this experiment.

**Table 8. Effect of each treatment group on volume, weight, and TGI of H292 subcutaneous xenograft tumors at the end of the experiment (D30)**

| Group | Dosing group | Tumor volume (mm³) | Tumor growth inhibition rate TGI (%) | Tumor weight (g) |
|---|---|---|---|---|
| Group 1 | PBS | 930.82 | N/A | 0.53 |
| Group 2 | 2 mg/kg EGFRxcMET-1 | 587.42* | 45% | 0.30** |
| Group 3 | 8 mg/kg EGFRxcMET-1 | 65.03**** | 113% | 0.03**** |
| Group 4 | 32 mg/kg EGFRxcMET-1 | 45.35**** | 115% | 0.01**** |

| | | | | |
|---|---|---|---|---|
| Note: compared to PBS group, "NS" P > 0.05; "*" P < 0.05; "**" P < 0.01; "****" P < 0.0001 | | | | |

### Example 6 Study on the Growth Inhibitory Effect of Combined Osimertinib on the Subcutaneous H1975-HGF Tumor Model in NOD/SCID Mice

In this study, NOD/SCID mice were subcutaneously inoculated with H1975-HGF (with high expression of EGFR (T790M/L858R), and c-MET) human lung cancer cells to establish a tumor model that can be used to evaluate the anti-tumor effect in relation to the mechanism of action of the test article and safety profile under disease conditions. This experiment were divided into 6 groups, with 6 mice in each group. PBS, 3 mg/kg Amivantamab (Rybrevant), 3 mg/kg EGFRxcMET-1, 3 mg/kg Osimertinib, 3 mg/kg Amivantamab combined with 3 mg/kg Osimertinib, 3 mg/kg EGFRxcMET-1 combined with 3 mg/kg Osimertinib were intraperitoneally administered, respectively. Osimertinib was administered once every day for a total of 6 doses; EGFRxcMET-1/Amivantamab was administered once every 2 days for a total of 2 doses. The growth inhibitory effect and safety of EGFRxcMET-1 combined with osimertinib on the Subcutaneous H1975-HGF tumor model in NOD/SCID mice were investigated.

Considering the results of tumor volume, TGI index and tumor weight at the endpoints of the trial, 1/6 tumors regressed in the EGFRxcMET-1 combined with osimertinib group, and no tumor regression was observed in other groups. The antitumor effect of EGFRxcMET-1 combined with osimertinib was significantly more potent than that of the two single drugs, showing a good combined synergistic effect. And the anti-tumor effect of EGFRxcMET-1 combined with osimertinib was better than that of Amivantamab combined with osimertinib.

**Table 9. Effects of each treatment group on volume, weight, and TGI of H1975-HGF tumors**

| Group | Dosing group | Tumor volume (mm³) | Tumor growth inhibition rate TGI (%) | Tumor weight (g) | Number of Tumor regression |
|---|---|---|---|---|---|
| Group 1 | PBS | 1619.09 | N/A | 1.12 | 0/6 |
| Group 2 | 3 mg/kg Amivantamab | 1026.62** | 49% | 0.79* | 0/6 |
| Group 3 | 3 mg/kg EGFRxcMET-1 | 593.88*** | 85% | 0.53*** | 0/6 |
| Group 4 | 3 mg/kg Osimertinib | 1030.39** | 49% | 0.84* | 0/6 |
| Group 5 | 3 mg/kg Amivantamab + 3 mg/kg Osimertinib | 483.72**** | 94% | 0.35**** | 0/6 |
| Group 6 | 3 mg/kg EGFRxcMET-1 + 3 mg/kg Osimertinib | 146.23**** | 122% | 0.10**** | 1/6 |

| | | | | | |
|---|---|---|---|---|---|
| Note: compared to PBS group, "NS" P > 0.05; "*" P < 0.05; "**" P < 0.01; "***" P < 0.001; "****" P < 0.0001; N/A, no statistical analysis was performed. | | | | | |

### Example 7 In Vivo Pharmacodynamic Study of Ba/F3 EGFR D770-N771 Ins_SVD Subcutaneous Xenograft Tumor Model in Nude Mice

In the mouse proB cell Ba/F3 EGFR D770-N771 ins_SVD subcutaneous xenograft tumor model of nude mouse containing EGFR exon 20 insertion mutation, 10 mg/kg Amivantamab, 3 mg/kg, 10 mg/kg EGFRxcMET-1 were administered intraperitoneally twice a week for 2 consecutive weeks (4 doses in total), and the corresponding tumor inhibition rates TGIs were 53.35%, 49.23%, and 58.41%, respectively. At the same dose (10 mg/kg), the tumor suppression effect of EGFRxcMET-1 was comparable to Amivantamab, and all animals were well tolerated.

**Table 10. Evaluation of anti-tumor efficacy in Ba/F3 EGFR-D770-N771ins_SVD xenograft tumor model**

| Group | Tumor volume (mm³) | T/C(%) | TGI(%) | *p*-value |
|---|---|---|---|---|
| Vehicle (solvent control) | 1003.01±64.41 | - | - | - |
| Human IgG Control | 1087.45±121.15 | 108.42 | -9.46 | - |
| Amivantamab (10 mg/kg) | 523.49±75.01 | 52.19 | 53.35 | <0.0001 |
| EGFRxcMET-1(3 mg/kg) | 559.94±35.20 | 55.83 | 49.23 | <0.0001 |
| EGFRxcMET-1(10 mg/kg) | 478.67±51.79 | 47.72 | 58.41 | <0.0001 |

### Example 8 In Vivo Pharmacodynamic Study of Ba/F3 EGFR-Del19/T790M/C797S Subcutaneous Xenograft Tumor Model in Nude Mice

In the mouse proB cell Ba/F3 EGFR-Del19/T790M/C797S subcutaneous xenograft tumor model of nude mouse containing EGFR triple-mutation, 10 mg/kg Amivantamab, 3 mg/kg, 10 mg/kg, and 30 mg/kg EGFRxcMET-1 were administered intraperitoneally twice a week for 2 consecutive weeks (4 doses in total), and the corresponding tumor inhibition rates TGIs were 97.17%, 75.81%, 96.57%, and 121.57%, respectively. The tumor suppression effect of EGFRxcMET-1 was significant and showed a good dose-response relationship. At the same dose (10 mg/kg), the tumor suppression effect of EGFRxcMET-1 was comparable to that of Amivantamab, and all animals were well tolerated.

**Table 11. Evaluation of anti-tumor efficacy in Ba/F3 EGFR-Del19/T790M/C797S xenograft tumor model**

| Group | Tumor volume (mm³) | T/C(%) | TGI(%) | *p*-value |
|---|---|---|---|---|
| Vehicle (solvent control) | 1164.27±113.75 | - | - | - |
| Human IgG Control | 1465.20±355.09 | 125.85 | -28.61 | - |
| Amivantamab (10 mg/kg) | 141.03±30.79 | 12.11 | 97.17 | <0.0001 |
| EGFRxcMET-1(3 mg/kg) | 366.75±45.00 | 31.50 | 75.81 | <0.0001 |
| EGFRxcMET-1(10 mg/kg) | 148.85±23.91 | 12.78 | 96.57 | <0.0001 |
| EGFRxcMET-1(30 mg/kg) | 87.35±9.81 | 7.50 | 121.57 | <0.0001 |

### Example 9 In Vivo Pharmacodynamic Study of Combined Almonertinib on Human Non-Small Cell Lung Cancer PC-9 Cell Line-Derived Subcutaneous Xenograft Tumor Model in Nude Mice

This study evaluated the synergistic antitumor effect of combination therapy with EGFRxc-Met bispecific antibody and almonertinib in a nude mouse subcutaneous xenograft tumor model derived from non-small cell lung cancer PC-9 cell line containing an EGFR exon 19 deletion mutation.

PC-9 cells were cultured in RPMI 1640 medium containing 10% fetal bovine serum and 1% penicillin and streptomycin in an incubator at 37°C, 5% CO2. After the cells were sufficiently aggregated, they were divided into bottles for passage, and the cells in the logarithmic growth phase were collected and counted. Cells were resuspended with PBS to a final concentration of 5.0 x 107 cells/mL for the inoculation. 0.1 mL of PC-9 cell suspension (containing 5 x 106 cells) was inoculated subcutaneously on the right dorsal region of 8-10 week BALB/c nude mice. The tumor growth was observed, and the animals were randomly divided into groups according to the tumor volumes and body weights. At the time of grouping, the average tumor volume was 328 mm3. The day of dosing was defined as the first day, i.e., Day 1. The remaining animals were euthanized at the end of the experiment. 5 groups were contained in this experiment, with one animal in each group. The experimental design was as follows:

**Table 12. Experimental design of PC-9 subcutaneous xenograft tumor model in nude mice**

| Group | Dosage (mg/kg) | Number of animals | Dosing volume (mL/kg) ^{a} | Route of administration^{b} | Frequency of administration^{c} |
|---|---|---|---|---|---|
| Vehicle (solvent control) | - | 10 | 10 | *i.p.* | biw x 4 w |
| IgG Control | 10 | 10 | 10 | *i.p.* | biw x 4 w |
| EGFRxcMET-1 | 10 | 10 | 10 | *i.p.* | biw x 8 w |
| Almonertinib | 10 | 10 | 10 | *p.o.* | qd x 90 d |
| EGFRxcMET-1 + Almonertinib | 10+10 | 10 | 10 | *i.p.*/ *p.o.* | biw/qd x 90 d |

| | | | | | |
|---|---|---|---|---|---|
| Administration volume: 10 L/g based on the body weight of the mouse. If weight loss exceeds 15%, the dosing regimen should be adjusted accordingly; p.o.: oral administration; i*.p.*: intraperitoneal administration; qd: once every day; biw: twice every week. | | | | | |

Mice in Vehicle solvent control and IgG control groups were euthanized on day 29 after continuous administration; Mice in EGFRxcMET-1 group were euthanized on day 60 after continuous administration; and mice in almonertinib group and the two-drug combination group were discontinued 90 days after continuous administration, and euthanasia was performed on day 65 after discontinuation. Tumor volume data at each time point for each group were presented as mean and standard error (SEM). Based on tumor volume data at different time points in each group, Dunnett's multiple comparisons test in Two-way or One-way ANOVA was used to analyze the differences between groups. All data analyses were performed with GraphPad Prism 9. p < 0.05 was considered statistically significant. The tumor inhibition rates of Day 22 after administration of each group are shown in Table 13, and the tumor inhibition rates of pharmacodynamic endpoints of each group are shown in Table 14.

**Table 13. Evaluation of antitumor efficacy of bispecific antibody combined with almonertinib in PC-9 xenograft tumor model (Day 22 after administration)**

| Group | Tumor volume (mm³) | T/C(%) | TGI(%) | *p*-value |
|---|---|---|---|---|
| Vehicle (solvent control) | 1510.99±154.57 | - | - | - |
| IgG Control | 1484.97±144.64 | 98.28 | 2.25 | - |
| EGFRxcMET-1 (10 mpk) | 184.00±25.71 | 12.18 | 112.32 | <0.0001 |
| Almonertinib (10 mpk) | 18.79±2.66 | 1.24 | 194.28 | <0.0001 |
| EGFRxcMET-1 + Almonertinib | 12.60±1.35 | 0.83 | 196.17 | <0.0001 |

| | | | | |
|---|---|---|---|---|
| Note: All data in the table are from day 22 | | | | |

**Table 14. Evaluation of antitumor efficacy of bispecific antibody combined with almonertinib in PC-9 xenograft tumor model (pharmacodynamic endpoint: day 155)**

| Group | Tumor volume (mm³) | T/C(%) | Tumor-bearing mice (number) | *p*-value |
|---|---|---|---|---|
| Vehicle (solvent control) | 2169.44±275.75 | - | 10/10 | - |
| Almonertinib (10 mpk) | 903.91±360.28 | 41.67 | 7/10 | <0.0001 |
| EGFRxcMET-1 + Almonertinib | 111.77±111.77 | 5.15 | 1/10 | <0.0001 |

| | | | | |
|---|---|---|---|---|
| Note: All data in the table are the efficacy endpoints on day 155 for each group | | | | |

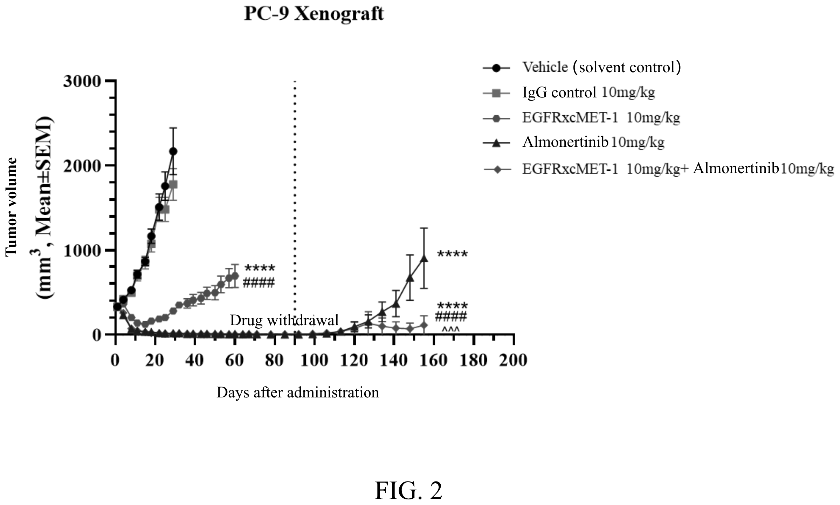

The efficacy evaluation of the almonertinib monotherapy group and the EGFRxcMET-1 + almonertinib combination group ended on Day155, and the tumor masses of mice were stripped and photographed. In the almonertinib monotherapy group, after 3 weeks of administration, the mean tumor volume was 18.79 mm3, and the TGI was 194.28%. And then, the mean tumor volume of the whole group continued to decrease. From day 78, the mean tumor volumes were less than 2 mm3, and tumors in 7 mice were completely regressed. After drug withdrawal, the regressed tumor in the almonertinib monotherapy group gradually recurred and continued to grow. The average tumor volume at the pharmacodynamic endpoint reached 903.91 mm3, and tumors in 4 of the 7 mice whose tumors had completely regressed showed regrowth; 7 mice were tumor-burdened at the pharmacodynamic endpoint (7/10). For the EGFRxcMET-1 + almonertinib combination group, after 3 weeks of continuous administration, the mean tumor volume was 12.60 mm³, and the TGI was 196.17%. And then, the mean tumor volume of the whole group continued to decrease. From day 78, the mean tumor volumes were less than 1 mm3, and tumors in 9 mice were completely regressed. After drug withdrawal, only tumors in 3 mice recurred, but their volumes remained below 5 mm3. At the pharmacodynamic endpoint, the average tumor volume of the combination group was 111.77 mm3, and only one mouse in the whole group was burdened with a tumor (1/10). The tumor volumes at different time points in each group are shown in FIG. 2.

EGFRxcMET-1 has a significant tumor suppression effect on subcutaneous xenograft tumor model derived from human non-small cell lung cancer PC-9 cell line containing EGFR exon 19 deletion mutation in nude mice. Compared with the EGFRxcMET-1 monotherapy group, the combination of EGFRxcMET-1 + almonertinib significantly enhanced the tumor suppression effect. Compared with the almonertinib monotherapy group, the combination of EGFRxcMET-1 + almonertinib significantly delayed the regrowth of tumors.

### Example 10 Phase I Clinical Study of Safety, Tolerability, Pharmacokinetics and Efficacy of the Egfrxc-Met Bispecific Antibody in Patients with Advanced Solid Tumors

### 1. Research purpose

To evaluate the safety and tolerability, PK profile, efficacy, immunogenicity, and other safety measures of EGFRxcMET-1 in subjects with locally advanced or metastatic NSCLC with EGFR mutations. To evaluate the efficacy, safety, PK (pharmacokinetic) profile, immunogenicity, and other efficacy measures of EGFRxcMET-1 in subjects with advanced solid tumors.

### 2. Test drug

The test drug was EGFRxcMET-1 injection, and the sequence was SEQ ID NOs: 21-24 in the present invention. Dosage form: injection. Specification: 10 mL: 200 mg/vial. The study drugs used in this experiment were supplied by Hansoh Pharma.

### 3. Dosing regimen

In the dose escalation experiment, the target subjects were treated with EGFRxcMET-1 intravenously. The dose escalation started from the preset starting dose of 400 mg and increased in the order of 400 mg, 800 mg, 1200 mg, 1600 mg, and 2000 mg.

Q2W group dosing regimen: subjects were administered once every week from Cycle 1 Day 1 (C1D1) and once every 2 weeks from Cycle 2 by intravenous drip, wherein every 4 weeks was a treatment cycle. In the 400 mg dose group, the first dose was administered on C1D1. In dose groups of 800 mg and more, the first dose was administered on two days of C1D1 and C1D2, wherein 400 mg was administered on C1D1, and the remainder of the dose was administered on C1D2. No divided administration is required for the second and subsequent doses.

Q3W Group Dosing Regimen: Subjects were administered intravenously, once every week on C1D1, C1D8, C1D15, and C2D1, followed by once every 3 weeks, and every 3 weeks was a treatment cycle. The first dose was administered on two days of C1D1 and C1D2, wherein 400 mg was administered on C1D1, and the remainder of the dose was administered on C1D2. No divided administration is required for the second and subsequent doses.

### 4. Inclusion criteria

Phase Ia: Subjects with histologically or cytologically confirmed locally advanced or metastatic NSCLC carrying an EGFR activating mutations, who had failed or were intolerant to or were unavailable to standard therapy.

Phase Ib: Cohort 1 included histologically or cytologically confirmed subjects with locally advanced or metastatic NSCLC carrying EGFR Exon 20ins mutations, who had failed or were intolerant to platinum-containing chemotherapy. Cohort 2 included other histologically or cytologically confirmed advanced solid tumors, who had failed or were intolerant of standard therapy.

### 5. Results of clinical trial

Of all 19 subjects enrolled, 14 subjects underwent dose escalation studies (1 subject in the 400 mg Q2W group, 3 subjects in the 800 mg Q2W group, 4 subjects in the 1200 mg Q2W group, and 6 subjects in the 1600 mg Q2W group), and 5 subjects underwent dose expansion studies (4 subjects in the 800 mg Q2W group, and 1 subject in the 2000 mg Q3W group).

Dose escalation was completed in 4 dose groups, and no dose-limiting toxicity DLT (dose-limiting toxicity) events were observed in any of them. The maximum tolerated dose MTD of EGFRxcMET-1 was not reached. Among the 16 subjects with evaluable efficacy, 1 subject experienced PR (partial response), 8 subjects experienced SD (stable disease, including 5 with reduced target lesions), and the DCR (disease control rate) was 56.3%.

In summary, the results of the current study showed that EGFRxcMET-1 had anti-tumor effects in subjects with advanced NSCLC carrying EGFR activating mutations who had failed or were intolerant to previous standard therapy, and preliminary anti-tumor activities were observed in all dose groups.

### Example 11 Phase Ib/III Clinical Study of Safety, Efficacy, Pharmacokinetics, and Immunogenicity of the Egfrxc-Met Bispecific Antibody Combined with Almonertinib Mesylate Tablets in Patients with Advanced Non-Squamous Non-Small Cell Lung Cancer

### 1. Research purpose

To evaluate the efficacy, safety, tolerability, PK profile, and immunogenicity of different dose levels of EGFRxcMET-1 in combination with almonertinib in subjects with advanced stage non-squamous NSCLC carrying EGFR mutations, who have not received systemic antitumor therapy.

### 2. Test drug

The test drug was EGFRxcMET-1 injection, and the sequence was SEQ ID NOs: 21-24 in the present invention. Dosage form: injection. Specification: 10 mL: 200 mg/vial. The study drugs used in this experiment were supplied by Hansoh Pharma.

The test drug was almonertinib mesylate tablets, dosage form: tablets, specification: 55 mg/tablet. The study drugs used in this experiment were supplied by Hansoh Pharma.

### 3. Dosing regimen

Two doses (800 mg; 1200 mg) of EGFRxcMET-1 injection were administered intravenously, once every week starting on C1D1 and every 2 weeks starting on Cycle 2, and every 4 weeks was a treatment cycle. The first dose was administered on two days of C1D1 and C1D2, wherein 400 mg was administered on C1D1, and the remainder of the dose was administered on C1D2. No divided administration is required for the second and subsequent doses.

From C1D1, almonertinib was administered orally at 110 mg once every day, and every 4 weeks was a treatment cycle.

### 4. Inclusion criteria

Histologically or cytologically confirmed locally advanced or metastatic non-squamous NSCLC, carrying EGFR-sensitive mutations (L858R mutation and/or exon 19 deletion mutation), either as single mutations or combined with other EGFR mutations. Subjects who had not received systemic antitumor therapy and pulmonary palliative therapy at the advanced stage.

While specific embodiments of the present invention have been described in detail, it will be understood by those skilled in the art that various modifications and variations may be made to the details in light of all the teachings that have been published and that such changes are within the scope of the present invention. The entirety of the present invention is given by the appended claims and any equivalents thereof.

## Claims

1. Use of a bispecific antibody in the manufacture of a medicament for preventing or treating a cancer, wherein the bispecific antibody comprises a first antibody or antigen-binding fragment against a first target c-Met, and a second antibody or antigen-binding fragment against a second target EGFR;
wherein the first antibody or antigen-binding fragment comprises an HCDR1 as set forth in SEQ ID NO: 01, an HCDR2 as set forth in SEQ ID NO: 02, and an HCDR3 as set forth in SEQ ID NO: 03; and, an LCDR1 as set forth in SEQ ID NO: 04, an LCDR2 as set forth in SEQ ID NO: 05, and an LCDR3 as set forth in SEQ ID NO: 06;
wherein the second antibody or antigen-binding fragment comprises an HCDR1 as set forth in SEQ ID NO: 09, an HCDR2 as set forth in SEQ ID NO: 10, an HCDR3 as set forth in SEQ ID NO: 11; and an LCDR1 as set forth in SEQ ID NO: 12, an LCDR2 as set forth in SEQ ID NO: 13, an LCDR3 as set forth in SEQ ID NO: 14.

2. The use according to claim 1, wherein in the bispecific antibody, the c-Met binding domain is a Fab region formed by a first heavy chain and a first light chain, the EGFR binding domain is a Fab region formed by a second heavy chain and a second light chain, and the first heavy chain and the second heavy chain bind to each other to form a heterodimeric Fc region.

3. Use of a bispecific antibody pharmaceutical composition in the manufacture of a medicament for preventing or treating a cancer, wherein the bispecific antibody pharmaceutical composition comprises the bispecific antibody defined in the use according to claim 1 or 2, and one or more pharmaceutically acceptable excipients, diluents, or carriers.

4. The use according to any one of claims 1 to 3, wherein the bispecific antibody or bispecific antibody pharmaceutical composition is used in combination with an EGFR inhibitor for the manufacture of the medicament.

5. The use according to claim 4, wherein the EGFR inhibitor is selected from one or more of almonertinib, erlotinib, gefitinib, befotertinib, osimertinib, furmonertinib, oritinib, Rezetinib, Avitinib, olmutinib, and rociletinib; and preferably is almonertinib or osimertinib.

6. The use according to any one of claims 1 to 5, wherein the first antibody or antigen-binding fragment comprises a heavy chain variable region as set forth in SEQ ID NO: 07 or having at least 90%, 91%, 92%, 93%, 94%, 95%, 96%, 97%, 98% or 99% identity to SEQ ID NO: 07, and a light chain variable region as set forth in SEQ ID NO: 08 or having at least 90%, 91%, 92%, 93%, 94%, 95%, 96%, 97%, 98% or 99% identity to SEQ ID NO: 08; and the second antibody or antigen-binding fragment comprises a heavy chain variable region as set forth in SEQ ID NO: 15 or having at least 90%, 91%, 92%, 93%, 94%, 95%, 96%, 97%, 98% or 99% identity to SEQ ID NO: 15, and a light chain variable region as set forth in SEQ ID NO: 16 or having at least 90%, 91%, 92%, 93%, 94%, 95%, 96%, 97%, 98% or 99% identity to SEQ ID NO: 16.

7. The use according to any one of claims 1 to 6, wherein the first antibody or antigen-binding fragment comprises a heavy chain as set forth in SEQ ID NO: 21 or having at least 90%, 91%, 92%, 93%, 94%, 95%, 96%, 97%, 98% or 99% identity to SEQ ID NO: 21, and a light chain as set forth in SEQ ID NO: 22 or having at least 90%, 91%, 92%, 93%, 94%, 95%, 96%, 97%, 98% or 99% identity to SEQ ID NO: 22; and the second antibody or antigen-binding fragment comprises a heavy chain as set forth in SEQ ID NO: 23 or having at least 90%, 91%, 92%, 93%, 94%, 95%, 96%, 97%, 98% or 99% identity to SEQ ID NO: 23, and a light chain as set forth in SEQ ID NO: 24 or having at least 90%, 91%, 92%, 93%, 94%, 95%, 96%, 97%, 98% or 99% identity to SEQ ID NO: 24.

8. The use according to any one of claims 1 to 7, wherein the bispecific antibody, the bispecific antibody pharmaceutical composition and the EGFR inhibitor are each comprised in different formulations as active ingredients, and are administered simultaneously, concurrently, sequentially, continuously, alternately, or separately.

9. The use according to any one of claims 1 to 8, wherein the cancer is selected from solid tumors, preferably advanced solid tumors.

10. The use according to any one of claims 1 to 9, wherein the cancer is a cancer expressing c-Met or/and EGFR.

11. The use according to claim 10, wherein the cancer expressing c-Met or/and EGFR is mediated by wild-type EGFR, EGFR activating mutations, EGFR gene amplification, increased level of circulating HGF, wild-type c-Met, c-Met activating mutations, c-Met gene amplification, MET gene amplification, or mutant KRAS, preferably mediated by EGFR activating mutations, or MET gene amplification.

12. The use according to claim 11, wherein the EGFR activating mutations comprise at least one mutation selected from the group consisting of: L718Q, L718V, G719A, G719S, G719C, G719D, L858R, L858P, L861Q, G724S, E746K, L747S, E749Q, A750P, A755V, V765M, C797S, S768I, or T790M substitutions; deletion of E746-A750; deletion of R748-P753; insertion of Ala (A) between M766 and A767; insertions of Ser, Val, and Ala (SVA) between S768 and V769; insertion of Asn and Ser (NS) between P772 and H773; insertion(s) of one or more amino acids between D761 and E762, between A763 and Y764, between Y764 and Y765, between M766 and A767, between A767 and V768, between S768 and V769, between V769 and D770, between D770 and N771, between N771 and P772, between P772 and H773, between H773 and V774, or between V774 and C775; deletion(s) of one or more amino acids in EGFR exon 19; or insertion(s) of one or more amino acids in EGFR exon 19; deletion(s) of one or more amino acids in EGFR exon 20; or insertion(s) of one or more amino acids in EGFR exon 20.

13. The use according to any one of claims 1 to 12, wherein the cancer is selected from an epithelial cell carcinoma, a breast cancer, an ovarian cancer, a lung cancer, a rectal cancer, an anal cancer, a prostate cancer, a kidney cancer, a bladder cancer, a head and neck cancer, a pharyngeal cancer, a nasal cancer, a pancreatic cancer, a skin cancer, an oral cancer, a tongue cancer, an esophageal cancer, a vaginal cancer, an endometrial cancer, a cervical cancer, a spleen cancer, a testicular cancer, a gastric cancer, a thymus cancer, a colon cancer, a thyroid cancer, a liver cancer or a melanoma, preferably a lung cancer or a liver cancer.

14. The use according to claim 12, wherein the cancer is a non-small cell lung cancer (NSCLC), a lung adenocarcinoma, a lung squamous cell carcinoma, a large cell lung cancer, a small cell lung cancer, a pulmonary sarcomatoid carcinoma, or a hepatocellular carcinoma (HCC);
preferably, the EGFR activating mutation is T790M/L858R, D770-N771 ins_SVD, Del19/T790M/C797S, or exon 19 deletion.

15. The use according to claim 14, wherein the cancer is a non-squamous non-small cell lung cancer.

16. The use according to any one of claims 4 to 15, wherein the EGFR inhibitor is administered in a dose of 10 to 500 mg, preferably 10 to 200 mg, more preferably 100 mg, 110 mg, 120 mg, 130 mg, 140 mg, 150 mg, 160 mg, 170 mg, 180 mg, 190 mg, or 200 mg.

17. The use according to any one of claims 4 to 16, wherein the EGFR inhibitor is administered at a frequency of twice a day, once a day, once every two days, or once every three days, preferably once a day, or once every two days.

18. The use according to any one of claims 3 to 17, wherein the bispecific antibody pharmaceutical composition has a unit concentration of about 1 mg/mL to about 100 mg/mL, preferably about 5 mg/mL to about 50 mg/mL, more preferably about 10 mg/mL to about 30 mg/mL; for example, 1, 5, 10, 15, 20, 25, 30, 35, 40, 45 or 50 mg/mL;
preferably, the bispecific antibody pharmaceutical composition further comprises a buffer and sucrose;
more preferably, the buffer is citric acid-sodium citrate with a concentration of 5 to 150 mM, for example 10 mM; and the concentration of sucrose is 20 to 60 mg/mL, for example 40 mg/mL.

19. The use according to any one of claims 3 to 18, wherein the bispecific antibody pharmaceutical composition is in a specification of 10 mL:10 mg to 10 mL:1000 mg, preferably 10 mL:100 mg to 10 mL:500 mg, more preferably 10 mL:100 mg, 10 mL:200 mg, 10 mL:300 mg, 10 mL:400 mg, or 10 mL:500 mg per vial.

20. The use according to any one of claims 1 to 19, wherein the bispecific antibody or the bispecific antibody pharmaceutical composition is administered at a dose of about 100 mg to about 5000 mg, preferably 400 mg to about 2000 mg, more preferably 400 mg, 800 mg, 1000 mg, 1200 mg, 1400 mg, 1600 mg, 1800 mg or 2000 mg.

21. The use according to any one of claims 1 to 20, wherein the bispecific antibody or the bispecific antibody pharmaceutical composition is administered at a frequency of once a day, once every two days, once every three days, twice a week, once a week, once every two weeks, once every three weeks, or once a month; preferably once a week, once every two weeks, or once every three weeks.

22. The use according to claim 21, wherein the bispecific antibody or the bispecific antibody pharmaceutical composition is administered at a frequency of:
1) once a week for a first treatment cycle, and once every two weeks starting from a second treatment cycle, wherein every four weeks is a treatment cycle; or
2) once a week for a first treatment cycle, and once every three weeks starting from a second treatment cycle, wherein every three weeks is a treatment cycle;
preferably, if a first dose is more than 400 mg, it is administered on the first and second days of the first cycle, with 400 mg administered on the first day, and the remainder of the dose administered on the second day; no divided administration is required for the second and subsequent doses.

23. A method of treating or preventing cancer, comprising administering the bispecific antibody or the bispecific antibody pharmaceutical composition defined in the use according to any one of claims 1 to 22 to a subject in need thereof;
preferably, the frequency of administration of the bispecific antibody or the bispecific antibody pharmaceutical composition is as defined in the use according to any one of claims 1 to 22; and/or the administered dose is as defined in the use according to claim 20.

24. The method according to claim 23, further comprising administering the EGFR inhibitor defined in the use according to any one of claims 4 to 17 in combination to a subject in need thereof, wherein EGFR inhibitor is administered simultaneously, concurrently, sequentially, continuously, alternately, or separately;
preferably, the frequency of administration of the EGFR inhibitor is as defined in the use according to any one of claims 17 to 22; and/or the administered dose is as defined in the use according to claim 16.

25. The method according to claim 23 or 24, wherein the cancer is a solid tumor; preferably an epithelial cell carcinoma, a breast cancer, an ovarian cancer, a lung cancer, a rectal cancer, an anal cancer, a prostate cancer, a kidney cancer, a bladder cancer, a head and neck cancer, a pharyngeal cancer, a nasal cancer, a pancreatic cancer, a skin cancer, an oral cancer, a tongue cancer, an esophageal cancer, a vaginal cancer, an endometrial cancer, a cervical cancer, a spleen cancer, a testicular cancer, a gastric cancer, a thymic cancer, a colon cancer, a thyroid cancer, a liver cancer or a melanoma; more preferably a non-small cell lung cancer, a lung adenocarcinoma, a lung squamous cell carcinoma, a large cell lung cancer, a small cell lung cancer, a pulmonary sarcomatoid carcinoma or a hepatocellular carcinoma.

26. A bispecific antibody pharmaceutical composition, wherein the bispecific antibody pharmaceutical composition is the bispecific antibody pharmaceutical composition defined in the use according to any one of claims 3 to 22;
preferably, the bispecific antibody pharmaceutical composition further comprises an EGFR inhibitor selected from one or more of almonertinib, erlotinib, gefitinib, befotertinib, osimertinib, furmonertinib, oritinib, Rezetinib, Avitinib, olmutinib, and rociletinib; and preferably almonertinib or osimertinib.
